# EUROPEAN PATENT APPLICATION

(11) **EP 2 946 793 A1**
(43) Date of publication of application: **25.11.2015**
(21) Application number: 14382182.5
(22) Date of filing: 23.05.2014
(51) Int. Cl.: A61K 47/48, A61P 35/00

(54) **Silicon particles targeting tumor cells**

(71) Applicant: Universitat Rovira I Virgili, 43003 Tarragona (ES); Fundacio Centre Tecnologic de la Quimica de Catalunya, 43007 Tarragona (ES); Institució Catalana De Recerca I Estudis Avançats (ICREA), 08010 Barcelona (ES); Universitat Politècnica de València, 46022 Valencia (ES); Consejo Superior de Investigaciones Científicas, 28006 Madrid (ES); Universidad de Vigo, 36310 Vigo - Pontevedra (ES); Fundación Hospital de Madrid, 28015 Madrid (ES); Philipps Universität Marburg, 35037 Marburg (DE); Medcomtech, S.A., 08840 Viladecans - Barcelona (ES)
(72) Inventor: Álvarez Puebla, Ramón Ángel, E-43007 Tarragona (ES); Fenollosa Esteve, Roberto, E-28049 Madrid (ES); Messeguer Rico, Francisco Javier, E-28049 Madrid (ES); Rodriguez, Marie Isabelle, E-28049 Madrid (ES); Álvarez Rodriguez, Rosana, E-36310 Vigo - Pontevedra (ES); Rodriguez de Lera, Ángel, E-36310 Vigo - Pontevedra (ES); Garcia-Rico Fernández, Eduardo, E-28015 Madrid (ES); Yu, Xiang, DE-35037 Marburg - Hessen (DE); Carregal Romero, Susana, DE-35037 Marburg - Hessen (DE); Parak, Wolfgang Johann, DE-35037 Marburg - Hessen (DE); Villanueva Leal, Carlos, 08840 Viladecans - Barcelona (ES); Rivera Gil, Pilar, 08840 Viladecans - Barcelona (ES); Álvarez Rodriguez, Susana, E-36310 Vigo - Pontevedra (ES)
(74) Representative: ABG Patentes, S.L.

(57) **Abstract**

A silicon particle comprising a silicon body, a functionalized silica surface surrounding the silicon body, and a targeting moiety specifically targeting tumor cells, and, optionally, an enzymatically metabolizable compound, is useful in the treatment of cancer by producing cell death after particle internalization.

## Description

### FIELD OF THE INVENTION

The invention relates to the field of cancer therapeutics using particles specifically targeting tumor cells and producing cell death after particle internalization.

### BACKGROUND OF THE INVENTION

Cancer is the second cause of death worldwide. In the case of breast cancer, epidemiological studies point to more than one million new cases diagnosed per year and an annual mortality rate close to 450,000 deaths.

Over the last two decades, nanoparticles have shown a great potential for drug delivery and cancer disease treatment. In most of these approaches, the nanoparticles are directed to the target cells by using antibodies attached to their surfaces, which in the case of *in vivo* administration supplement passive targeting through the enhanced permeability and retention (EPR) effect. Some strategies concern heating nanoparticles with an external oscillating magnetic or electromagnetic field and killing the nearby cells through magnetothermia or photothermia. Although these approaches are already in clinical use, some cytotoxic effects have been reported. However, these materials suffer from certain limitations such as the large and expensive facilities (i.e., magnetic resonance imaging or "MRI") necessary for magnetothermia therapy and the limited penetration depth of light in the body, bioaccumulation effects and finally intercalation effects on DNA, in the case of photothermia.

Other approaches make use of antibody-functionalized nanoparticles loaded with a cancer drug for its delivery to tumoral cells. In this context, porous silicon nanoparticles (PSiNPs) have been considered as a very promising platform for cancer therapy because of their excellent biocompatibility and biodegradability properties.

However, in all the reported studies, PSiNPs work either as a passive platform, that carry the anticancer load, or as an active element after activation with the appropriate trigger, namely light and acoustic waves for particle thermalization, or singlet oxygen generation in photodynamic therapies.

Therefore, there is still a need to develop effective anti-tumoral drugs alternatives, such as nano- or micro-technology-based antitumoral agents, that overcome the disadvantages of the agents described so far in the art.

### SUMMARY OF THE INVENTION

The authors of the present invention have developed a silicon particle working itself as an efficient and selective cancer cell killer. Silicon is characterized by a low reduction potential which, in the case of silicon particles (SiPs), in general, promotes explosive oxidation reactions with energy yield exceeding that of TNT (trinitrotoluene) when exposed to water. A previous exposition of SiPs to open atmosphere generates a thin layer of water soluble silicon dioxide (SiO₂) or silica. Functionalization of the silica layer with a suitable reagent, such as an enzymatically metabolizable compound (e.g., a sugar), quenches its solubility. By further functionalization with an appropriate agent, such as targeting moiety (e.g., an antibody), it is possible to increase SiPs bioaccumulation inside the target cells by using the cellular enzymatic machinery to metabolize the enzymatically metabolizable compound, exposing first the water soluble silica surfaces to water and then the silicon (Si), which reacts violently and produces target cell death. Additionally, this explosive reaction yields soluble biocompatible residues, which are easily excretable by urine.

Thus, in an aspect, the invention relates to a silicon particle selected from the group consisting of a type I silicon particle and a type II silicon particle, wherein the type I silicon particle comprises:
a. a silicon body,
b. a silica surface surrounding the silicon body, wherein the silica surface is functionalized by at least one functional group, and wherein said functional group is capable of setting up a bond with an enzymatically metabolizable compound,
c. an enzymatically metabolizable compound, wherein said compound is linked to (i) said functionalized silica surface through said functional group and to (ii) a targeting moiety, and
d. a targeting moiety, wherein said moiety is linked to the particle through said enzymatically metabolizable compound,
and wherein the type II silicon particle comprises:
a. a silicon body,
b. a silica surface surrounding the silicon body, wherein the silica surface is functionalized by at least one functional group, and wherein said functional group is capable of setting up a bond with a targeting moiety, and
c. a targeting moiety, wherein said moiety is directly linked to said functionalized silica surface.

In another aspect, the invention relates to a process for producing the above mentioned silicon type I silicon particle, which comprises:
i) contacting a silicon particle comprising a silicon body and a functionalized silica surface with an enzymatically metabolizable compound under conditions suitable for binding the compound to the functionalized silica surface; and
ii) contacting the product resulting from step i) with a targeting moiety under conditions suitable for binding the targeting moiety to the compound bound to the functionalized silica surface.

In another aspect, the invention relates to a process for producing the above mentioned silicon type II silicon particle, which comprises contacting a silicon particle comprising a silicon body and a functionalized silica surface with a targeting moiety under conditions suitable for binding the targeting moiety to the functionalized silica surface.

In a further aspect, the invention relates to a composition comprising at least said silicon particle.

In a further aspect, the invention relates to a pharmaceutical composition comprising a therapeutically effective amount of said silicon particle and a pharmaceutically acceptable excipient.

In a further aspect, the invention relates to said silicon particle, composition or pharmaceutical composition comprising said silicon particle, for use in medicine.

In a further aspect, the invention relates to said silicon particle, composition or pharmaceutical composition comprising said silicon particle, for use in the prevention and/or treatment of cancer.

### BRIEF DESCRIPTION OF THE FIGURES

**Figure 1** shows a representative transmission electron microscopy (TEM) image of the silicon particles according to the invention.
**Figure 2** shows a synthetic scheme for the sugar synthesis and derivation, and for the antibody coupling to the particles of the invention.
**Figure 3** shows a relative cell viability plot after incubation of human breast cancer cell line SK-BR-3 cells (a), which overexpress HER2, and of human breast cancer cell line MDA-MB-435 cells (b) with PSiPs and PSiPs-HER2 during 48 h.

### DETAILED DESCRIPTION OF THE INVENTION

### 1. Definitions

The term "antibody" (abbreviated Ab), also known as immunoglobulin, (abbreviated Ig), as used herein, relates to immunoglobulin molecules and immunologically active portions of immunoglobulin molecules, i.e., molecules containing an antigen binding site, which specifically bind (immunoreact) with an antigen, such as, for example, a protein. Antibodies according to the invention include any agent capable of binding to a ligand with high affinity, including IgG, IgM, IgA, IgD and IgE, as well as molecules similar to antibodies which have an antigen binding site, such as Fab', Fab, F(ab')2, single domain antibodies or DABS, Fv, scFv and the like, also known as "antigen binding fragments of an antibody". The techniques for preparing said antibodies are very well-known for the person skilled in the art and include the methods described by Ausubel et al. (Current Protocols in Molecular Biology, eds. Ausubel et al. John Wiley & Sons 1992).

The term "antigen", as used herein, relates to any molecule or molecular fragment thereof that, when introduced into the body, induces a specific immune response (i.e. humoral or cellular) by the immune system. Antigens have the ability to be bound at the antigen-binding site of an antibody.

The term "aptamer", as used herein, relates to a short variable peptide or nucleic acid domain that binds to a specific target molecule. Aptamers are usually created by selecting them from a large random sequence pool, but natural aptamers also exist in riboswitches. Aptamers can be used for both basic research and clinical purposes as macromolecular drugs. The variable loop length is typically composed of ten to twenty amino acids, and the scaffold may be any protein which has good solubility and compacity properties. Currently, the bacterial protein Thioredoxin-A is the most used scaffold protein, the variable loop being inserted within the reducing active site, which is a Cys-Gly-Pro-Cys loop in the wild protein, the two Cys lateral chains being able to form a disulfide bridge. Peptide aptamer selection can be made using different systems, including the yeast two-hybrid system, phage display, mRNA display, ribosome display, bacterial display and yeast display.

The term "bond" or "chemical bond", as used herein, relates to the attraction between atoms allowing the formation of chemical substances that contain two or more atoms. The strength of chemical bonds varies considerably: there are "strong bonds", including covalent and ionic bonds, and "weak bonds", including dipole-dipole interactions, the London dispersion force and hydrogen bonding.

The term "binding structure", as used herein, relates to a molecule that is able to specifically interact with (a) potential binding partner(s) so that it is able to discriminate between said potential binding partner(s) and a plurality of different molecules as said potential binding partner(s) to such an extent that, from a pool of said plurality of different molecules as potential binding partner(s), only said potential binding partner(s) is/are bound, or is/are significantly bound. Methods for the measurement of binding of a binding structure to a potential binding partner are known in the art and can be routinely performed e.g. by using ELISA, isothermal titration calorimetry, equilibrium dialysis, pull down assays or a Biacore apparatus. Exemplary binding structures which are useful in the context of the present invention include, but are not limited to antibodies, antibody fragments such as Fab fragments, F(ab')2 fragments, single chain variable fragments (scFv), isolated variable regions of antibodies (VL- and/or VH-regions), CDRs, single domain antibodies, CDR-derived peptidomimetics, lectins, lipocalins or various types of scaffold-derived binding structures as described, for example, in Skerra 2000 J Mol Recognit 13:167-187 or Binz 2005 Nat Biotechnol 23:1257-1268. A binding structure can bind to, for example, a DNA molecule (a DNA-binding structure), an RNA molecule (an RNA-binding structure) and/or a protein molecule (a protein-binding structure). In the case of a protein-binding structure, it can bind to itself (to form homodimers, homotrimers, etc.) and/or it can bind to one or more molecules of a different protein or proteins. A binding structure can have more than one type of binding activity. For example, zinc finger proteins have DNA-binding, RNA-binding and protein-binding activity.

The term "cancer" or "tumor" or "tumor disease" or "neoplasm", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. Cancers usually share some of the following characteristics: sustaining proliferative signalling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumour cells resemble, which is therefore presumed to be the origin of the tumour. These types include:
- Carcinoma: Cancers derived from epithelial cells. This group includes many of the most common cancers, particularly in the aged, and include nearly all those developing in the breast, prostate, lung, pancreas, and colon.
- Sarcoma: Cancers arising from connective tissue (i.e. bone, cartilage, fat, nerve), each of which develop from cells originating in mesenchymal cells outside the bone marrow.
- Lymphoma and leukaemia: These two classes of cancer arise from hematopoietic (blood-forming) cells that leave the marrow and tend to mature in the lymph nodes and blood, respectively. Leukaemia is the most common type of cancer in children accounting for about 30%.
- Germ cell tumour: Cancers derived from pluripotent cells, most often presenting in the testicle or the ovary (seminoma and dysgerminoma, respectively).
- Blastoma: Cancers derived from immature "precursor" cells or embryonic tissue. Blastomas are more common in children than in older adults.

The term "carbohydrate", also known as "saccharide" or "sugar", refers to a macromolecule of carbon, hydrogen and oxygen and includes monosaccharides, disaccharides, oligosaccharides, and polysaccharides. The term "monosaccharide", as used herein, refers to a simple form of a sugar that consists of a single saccharide unit which cannot be further decomposed to smaller saccharide building blocks or moieties, including without limitation furanose, fructose, glucose, galactose, mannose, a modified monosaccharide, sialic acid and eritrose and mixtures thereof. The monosaccharides may be in its lineal or cyclic forms (hemiacetalic cyclic isomers). The furanose is any simple sugar containing a five-membered furan-based ring, such as a D-ribose or a fructose residue (D-(-)-fructofuranose). With the combination of the monosaccharides, multiple sugar structures can be attained.

The term "composition", as used herein, relates to a material composition that comprises at least two components, as well as any product resulting, directly or indirectly, from the combination of the different components in any quantity thereof. Those skilled in the art will observe that the composition may be formulated as a single formulation or may be presented as separate formulations of each of the components, which may be combined for joint use as a combined preparation. The composition may be a kit-of-parts wherein each of the components is individually formulated and packaged.

The term "enzymatically metabolizable compound", as used herein, relates to any compound that is susceptible of being metabolized by a cellular enzyme, i.e., the metabolism of the enzymatically metabolizable compound takes place within the target cell. Enzymatically metabolized compounds for use within the context of the present invention include, without limitation, carbohydrates, lipids, peptides, proteins, and nucleic acids. In a particular embodiment, the enzymatically metabolized compound of the silicon particles of the invention is a carbohydrate, more particularly a pyranose or a glucopyranoside.

The term "enzyme", as used herein, refers to a biological macromolecule that functions as a highly selective catalyst, greatly accelerating both the rate and specificity of a metabolic reaction for which it is specific.

The term "functional group", as used herein, relates to a specific group of atoms or bonds within molecules that is responsible for the characteristic chemical reactions of said molecules. The same functional group undergoes the same or similar chemical reaction(s) regardless of the size of the molecule it is a part of. However, its relative reactivity can be modified by nearby functional groups.

The term "glucopyranoside", as used herein, relates to a carbohydrate having a chemical structure that includes a six-membered ring comprising five carbon atoms and one oxygen atom, and derivatives thereof.

The term "lipid" encompasses both naturally occurring and synthetically produced lipids and includes, without limitation, fatty acids; fats; oils; waxes; cholesterol; sterols; fat-soluble vitamins, such as vitamins A, D, E and K; monoglycerides; diglycerides, and phospholipids. Preferred fatty acids include lauroic acid (C12), myristic acid (C14), palmitic acid (C16), stearic acid (C18), and docosanoic acid (C22).

The term "nucleic acid", as used herein, refers to a polymer of nucleotides having two or more deoxyribonucleotide, ribonucleotide or nucleotide analog molecules as well as molecules that are structurally similar to a native nucleic acid, but differ from the native nucleic acid (e.g. through chemical modification) at one or more of the nucleic acid backbone (e.g. phosphate in native nucleic acids), nucleic acid sugar (e.g. deoxyribose for native DNA and ribose in native RNA), and nucleic acid base (e.g. adenosine, cytosine, guanine or thymidine in native nucleic acids). The nucleic acid can be a double stranded or single stranded nucleic acid including, without limitation, DNA, RNA, oligonucleotides, PNAs, cDNA, RNAi, shRNA, miRNA, siRNA, ribozymes, antisense oligonucleotides, as well as modified forms thereof. In a preferred embodiment the compound is a nucleic acid.

The term "particle", as used herein, relates to silicon particles both in the micromolar (microparticles) and the nanomolar (nanoparticles) range. In particular, nanoparticles are structures with a mean particle size generally comprised between 20 and 500 nm.

The term "peptide" or "polypeptide", as used herein, refers to a short polymer of amino acid monomers linked by peptide bonds, typically containing less than 50 monomer units. Any peptide may be used in the present invention.

The term "protein", as used herein, refers to one or more peptides (or polypeptides), i.e., polymer chains of amino acids bonded together by peptide bonds between the carboxyl and amino groups of adjacent amino acid residues, optionally including modifications, e.g., post-translational modifications, which alter the physical and chemical properties, folding, stability, activity, and ultimately, the function of the proteins. Proteins having non-peptide groups attached (i.e., prosthetic groups or cofactors) are also included within this definition. The number of amino acid residues in a protein can vary in a broad range, for example, the polymer chain of amino acid residues linked by peptide bonds may contain typically 50 or more amino acids residues.

The term "pharmaceutical composition", as used herein, relates to a composition comprising at least a particle provided by the present invention together with a pharmaceutically acceptable carrier.

The terms "pharmaceutically acceptable vehicle", "pharmaceutically acceptable carrier," "pharmaceutically acceptable diluent" or "pharmaceutically acceptable excipient", used interchangeably herein, refer to a non-toxic solid, semisolid or liquid filler, diluent, encapsulating material or formulation auxiliary of any conventional type. A pharmaceutically acceptable carrier is essentially non-toxic to recipients at the employed dosages and concentrations and is compatible with other ingredients of the formulation. The number and the nature of the pharmaceutically acceptable carriers depend on the desired administration form. The pharmaceutically acceptable carriers are known and may be prepared by methods well known in the art [see, for example, Faulí i Trillo C, "Tratado de Farmacia Galénica" (Ed. Luzán 5, S.A., Madrid, ES, 1993) and Gennaro A, Ed., "Remington: The Science and Practice of Pharmacy" 20th Ed. (Lippincott Williams & Wilkins, Philadelphia, PA, US, 2003)]. They are involved in carrying or transporting the subject chemical from one organ, or portion of the body, to another organ, or portion of the body without excessive toxicity, irritation, allergic response, or other problem or complication, commensurate with a reasonable benefit/risk ratio. Each carrier must be "acceptable" in the sense of being compatible with the other ingredients of the formulation and not injurious to the patient. Some examples of materials which can serve as pharmaceutically-acceptable carriers include: (a) sugars (e.g. lactose, glucose and sucrose), (b) starches (e.g. corn starch and potato starch), (c) cellulose and its derivatives (e.g. sodium carboxymethyl cellulose, ethyl cellulose and cellulose acetate), (d) powdered tragacanth, (e) malt, (f) gelatin, (g) talc, (h) excipients (e.g. cocoa butter and suppository waxes), (i) oils (e.g. peanut oil, cottonseed oil, safflower oil, sesame oil, olive oil, corn oil and soybean oil), (j) glycols (e.g. propylene glycol), (k) polyols (e.g. glycerin, sorbitol, mannitol and polyethylene glycol), (1) esters (e.g. ethyl oleate and ethyl laurate), (m) agar, (n) buffering agents (e.g. magnesium hydroxide and aluminum hydroxide), (o) alginic acid, (p) pyrogen-free water, (q) isotonic saline, (r) Ringer's solution, (s) ethyl alcohol, (t) phosphate buffer solutions and (u) other non-toxic compatible substances employed in pharmaceutical formulations. Wetting agents, emulsifiers and lubricants (e.g. sodium lauryl sulfate and magnesium stearate), as well as coloring agents, release agents, coating agents, sweetening, flavoring and perfuming agents, preservatives and antioxidants can also be present in the compositions. Examples of pharmaceutically-acceptable antioxidants include: (a) water soluble antioxidants (e.g. ascorbic acid, cysteine hydrochloride, sodium bisulfate, sodium metabisulfite or sodium sulfite), (b) oil-soluble antioxidants (e.g. ascorbyl palmitate, butylated hydroxyanisole (BHA), butylated hydroxytoluene (BHT), lecithin, propyl gallate or α-tocopherol), and (c) metal chelating agents (e.g. citric acid, ethylenediamine tetraacetic acid (EDTA), sorbitol, tartaric acid or phosphoric acid).

The term "prevention", "preventing" or "prevent", as used herein, relates to the administration of the particle according to the invention or of a medicament comprising said particle to a subject in an initial or early stage of a disease, in particular cancer, or also to avoid the appearance of said disease. The prevention may be complete (e.g. the total absence of a disease). The prevention may also be partial, such that for example the occurrence of a disease in a subject is less than that which would have occurred without the present invention. Prevention also refers to reduced susceptibility to a clinical condition.

The term "silica" or "silicon oxide" or "silicon dioxide", as used herein, relates to a compound of formula SiO₂.

The term "silicon", as used herein, relates to a chemical element with the symbol Si and atomic number 14.

The term "small molecule", as used herein, relates to a low molecular weight [i.e., equal to or less than (≤) 900 Daltons] organic compound that may help regulate a biological process, with a size on the order of 10⁻⁹ m. Most drugs are small molecules. The upper molecular weight limit for a small molecule is approximately 900 Daltons, which allows for the possibility to rapidly diffuse across cell membranes so that they can reach intracellular sites of action. In addition, this molecular weight cutoff is a necessary but insufficient condition for oral bioavailability. A lower molecular weight cutoff of 500 Daltons has been recommended for small molecule drug development candidates based on the observation that clinical attrition rates are significantly reduced if the molecular weight is kept below this 500 Dalton limit. Small molecules can have a variety of biological functions, serving as cell signaling molecules, as drugs in medicine, as pesticides in farming, and in many other roles. These compounds can be natural, such as secondary metabolites, for example, alkaloids, glycosides, lipids, nonribosolmal peptides (e.g., actinomycin-D), phenazines, natural phenols (including flavonoids), polyketide, terpenes (including steroids), tetrapyrroles, etc.), or artificial (such as some drugs, i.e., chemically derived, man-made compounds developed to treat a wide range of diseases); they may have a beneficial effect against a disease (such as drugs) or may be detrimental (such as teratogens and carcinogens). Biopolymers such as nucleic acids, proteins, and polysaccharides (such as starch or cellulose) are not small molecules, although their constituent monomers, ribo- or deoxyribo-nucleotides, amino acids, and monosaccharides, respectively, are often considered small molecules. Very small oligomers are also usually considered small molecules, such as dinucleotides, peptides such as the antioxidant glutathione, and disaccharides such as sucrose. In a particular embodiment, the small molecule is a molecule that binds to a specific biopolymer, such as a protein or a nucleic acid, and acts as an effector, altering the activity or function of the biopolymer. Further, small molecules may also be used as research tools to probe biological function as well as leads in the development of new therapeutic agents. Some can inhibit a specific function of a multifunctional protein or disrupt protein-protein interactions. In a particular embodiment, the small molecule is folic acid. The term "folic acid", or "folate" or "folacin" or "folic acid" or "vitamin B9", as used herein, relates to (2S)-2-[(4-{[(2-amino-4-hydroxypteridin-6-yl)methyl]amino} phenyl)formamido]pentanedioic acid, a water soluble vitamin that belongs to the B-complex group of vitamins.

The term "subject" or "individual" or "animal" or "patient" includes any subject, particularly a mammalian subject, for whom therapy is desired. Mammalian subjects include humans, domestic animals, farm animals, and zoo or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human of any race and sex.

The term "targeting moiety", as used herein, relates to a functional group which serves to target or direct the particle of the invention to a particular location, cell type, diseased tissue, or association. In general, the targeting moiety is directed against a target molecule and allows concentration of the particles in a particular localization within a subject. In a non-exhaustive list, antibodies, cell surface receptor ligands and hormones, lipids, sugars and dextrans, alcohols, bile acids, fatty acids, amino acids, peptides and nucleic acids may all be attached to localize or target the particles of the invention to a particular site. In a particular embodiment, the targeting moiety allows targeting the particles of the invention to a particular tissue or to the surface of a target cell, particularly a tumoral cell. In a particular embodiment, the targeting moiety comprises an antibody or an antigen binding fragment thereof, a binding structure, an aptamer, a peptide or a small molecule. More particularly, the targeting moiety is an antibody or an antigen binding fragment thereof.

The term "therapeutically effective amount", as used herein in relation to the silicon particle of the invention, or in relation to the agent, excipient and/or carrier comprised by the pharmaceutical composition of the invention, relates to the sufficient amount of said particle, agent, excipient and/or carrier to provide the desired effect, i.e. to achieve an appreciable prevention, cure, delay, reduction of severity or amelioration of one or more symptoms and signs derived from a disease, and will generally be determined by, among other causes, the characteristics of the agent itself and the therapeutic effect to be achieved. It will also depend on the subject to be treated, the severity of the disease suffered by said subject, the chosen dosage form, etc. For this reason, the doses mentioned in this invention must be considered only as guides for the person skilled in the art, who must adjust the doses depending on the aforementioned variables. In an embodiment, the effective amount produces the amelioration of one or more symptoms of the disease that is being treated.

The term "therapy", as used herein, refers to the attempted remediation of a health problem, usually following a diagnosis, or to prevention or the appearance of a health problem. As such, it is not necessarily a cure, i.e. a complete reversion of a disease. Said therapy may or may not be known to have a positive effect on a particular disease. This term includes both therapeutic treatment and prophylactic or preventative measures, in which the object is to prevent or stop (reduce) an undesired physiological change or disorder, such as, cancer. For the purpose of this invention, beneficial or desired clinical results include, without limitation, relieving symptoms, reducing the spread of the disease, stabilizing pathological state (specifically not worsening), slowing down or stopping the progression of the disease, improving or mitigating the pathological state and remission (both partial and complete), both detectable and undetectable. It can also involve prolonging survival, disease free survival and symptom free survival, in comparison with the expected survival if treatment is not received. Those subjects needing treatment include those subjects already suffering the condition or disorder, as well as those with the tendency to suffer the condition or disorder or those in which the condition or disorder must be prevented.

The term "treatment", as used herein, relates to both therapeutic measures and prophylactic or preventative measures, wherein the object is to prevent or slow down (lessen) an undesired physiological change or disorder, such as cancer. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. Treatment also means prolonging survival as compared to expected survival if not receiving treatment. Those in need of treatment include those already with the condition or disorder as well as those prone to have the condition or disorder or those in which the condition or disorder is to be prevented.

### 2. Silicon particles of the invention

The authors of the present invention have developed silicon particles with therapeutic applications based on their capacity to specific target cells, such as tumor cells. The silicon particles provided by the present invention are generated as silicon particles, wherein their exposition to atmospheric oxygen generates a thin layer of silica (SiO₂). This SiO₂ layer protects the silicon (Si) body thus acting as a protective shell and also providing an easily functionalizable surface to couple an organic layer that protects silica from dissolution in physiological media. Additionally, this protective shell may be easily degradable by the enzymatic machinery of a cell. In a particular embodiment, inventors have used glucopyranoside, a well-known molecule which can be easily metabolized by the lysosomal enzyme α-D-glucoside glucohydrolase. The lysosome has been shown to be a target organelle for most particles. Said particles may be targeted via a targeting moiety or vector to a specific target cell, such as a tumor cell overexpressing a cell receptor. In particular, the HER2/neu (CD340/p185) positive breast cancer is characterized by the amplification of the gene ERBB2 and its protein (HER2/neu) high expression and activity. In fact, there is a strong association between HER2/neu (ErbB2) tyrosine kinase expression and the aggressiveness and prognosis of the disease. Inventors have developed, in a particular embodiment, particles comprising a silicon body and a silica surface surrounding the silicon body, wherein said silica surface has been further functionalized and at least partially coated with glucopyranoside, and wherein an antibody to HER2 has been attached to say particles based on its affinity for that sugar. Said particles are internalized by the target cells, e.g., tumoral cells, causing specific cell death (see Example 2). Advantageously, cell death yields soluble biocompatible residues which are easily excretable by urine.

Thus, in an aspect, the present invention relates to a silicon particle, hereinafter referred to as the "particle of the invention", selected from the group consisting of a type I silicon particle and a type II silicon particle, wherein the type I silicon particle comprises:
a. a silicon body,
b. a silica surface surrounding the silicon body, wherein the silica surface is functionalized by at least one functional group, and wherein said functional group is capable of setting up a bond with an enzymatically metabolizable compound,
c. an enzymatically metabolizable compound, wherein said compound is linked to (i) said functionalized silica surface through said functional group and to (ii) a targeting moiety, and
d. a targeting moiety, wherein said moiety is linked to the particle through said enzymatically metabolizable compound,
and wherein the type II silicon particle comprises:
a. a silicon body,
b. a silica surface surrounding the silicon body, wherein the silica surface is functionalized by at least one functional group, and wherein said functional group is capable of setting up a bond with a targeting moiety, and
c. a targeting moiety, wherein said moiety is directly linked to said functionalized silica surface.

In a particular embodiment, the mean size of the silicon particle of the invention ranges from 20 nm to 5 µm, more particularly from 50 to 500 nm, still more preferably from 100 to 200 nm. The term "average size" or "mean size", as used herein, relates to the average diameter of a population of particles moving together in a medium. The average size of these systems can be measured by standard processes known by persons skilled in the art including, without limitation, dynamic light scattering (DLS) and asymmetric flow field flow fractionation (AFFFF) using multi-angle light scattering (MALS) or electronic microscopy. The average size of the particles can be mainly affected by the amount and molecular weight of the enzymatically metabolizable compound, and by the nature and amount of the targeting moiety present in the particles of the invention (generally, the larger the amount or molecular weight of said components, the larger the average size of the particle), and by some parameters of the process for the production of said particles, such as the stirring speed, etc.

### 2.1 Silicon type I particles

### 2.1.1 Silicon body (core)

The silicon type I particle of the invention comprises a silicon body (also known as the "core" of the particle of the invention).

In a particular embodiment, the silicon body of the type I particle of the invention comprises a solid silicon body.

In another particular embodiment, the silicon body of the type I particle of the invention does not comprise any pores. In another embodiment, the silicon body of the type I particle of the invention comprises one or more pores. The size of the pores of the silicon body eventually present in the particle of the invention is included within the nanomolar range, preferably about 1-2 nm. Methods to determine pore size are known for the skilled person and include, without limitation, transmission electron microscopy (TEM), gas adsorption isotherms or adsorption in liquid or gas phase of molecules of variable size.

Methods for obtaining silicon particles are known and include, without limitation, the methods described in WO2012101306, Harris JT et al. 2010 Chem Mater 22: 6378-6383 and Shi L et al. 2013 Nat Commun 4: 1904). In a particular embodiment, small silicon particles (smaller than 400 nm) are obtained through mechanical milling of silicon powder together with a further particle separation through a colloidal sedimentation process. The larger a-Si:H particles (380-650 nm) are prepared by trisilane (Si₃H₈; 100%, Voltaix) decomposition in supercritical n-hexane (95%, anhydrous, Sigma-Aldrich) in a titanium reactor with the internal volume of 10 mL. The a-Si:H particle size is adjusted by changing the amount of hexane and trisilane used in the reaction. In a particular embodiment, a-Si:H particles with 380 nm diameter and 50-60 at.% H-content are made by loading 6.4 mL of n-hexane and 10 µL of trisilane into a cylindrical titanium reactor with 10 mL internal volume in a nitrogen-filled glovebox. The reactor is sealed and removed from the glovebox. A brass heating block is preheated to 50°C above the desired reaction temperature. For instance, for a reaction run at 420°C, the heating block is preheated to 470°C. After placing the reactor into the preheated block, the temperature typically drops by about 40°C below the desired reaction temperature and returns to the reaction temperature in 3 min. After 10 min of total heating time, the reactor is removed from the heating block and submerged in an ice bath. After cooling to room temperature, the reactor is opened to collect the product with 5 mL of chloroform. The product is centrifuged at 8000 rpm for 5 min to precipitate the particles. The supernatant is discarded. The particles are dispersed in 2 mL of chloroform and stored in air. A second step concerns submitting particles to a vacuum annealing treatment to obtain high refractive index SCs. X-ray diffraction and Raman spectroscopy showed that the as-synthesized colloids are made of hydrogenated amorphous silicon. Then monodisperse particles are submitted to an annealing process at 500°C for removing hydrogen, thus increasing the refractive index of particles up to a value equivalent to the 90% of the refractive index of bulk silicon.

### 2.1.2 Particle silica surface (shell)

The type I silicon particle of the invention further comprises a silica surface, also referred to as the "shell" of the particle, wherein said silica surface is located surrounding the silicon body or core of the particle.

The silica surface of the type I silicon particle of the invention is functionalized by at least one functional group, wherein said functional group is capable of setting up a bond with an enzymatically metabolizable compound. Functional groups for use within the context of the invention include those resulting from the reaction of silica (surface of the particle of the invention) with a functionalizing agent such as a silane, wherein said silane comprises a group for functionalizing, and include without limitation halosilanes, organosilanes, silanoles, siloxanes, aminosilanes, mercaptosilanes and glycidoxysilanes. The term "halosilane" refers to any halogen substituted silane and includes, without limitation, octadeciltriclorosilane (OTS), diclorometilsilane (DCDMS), trimethylchlorosilane, cynanoproyldimethyl-chlorosilane, phenyldimethylchlorosilane, chloromethyldimethylchlorosilane, (trideca-fluoro-1,1,2,2-tetrahydro-octyl)dimethyl chlorosilane, n-octyldimethylchlorosilane, and n-octadecyldimethyl chlorosilane. The term "organosilane" refers to any organic derivative of a silane containing at least one carbon to silicon bond and includes, without limitation, 3-aminopropyltriethoxysilane, N-propyltrichlorosilane, dimethylphenylsilane, and 3-cyanopropyltrichlorosilane. The term "silanol" refers to any hydroxy derivative of a silane and includes, without limitation, alkoxysilanols, alkoxyalkylsilanols, alkoxysilanediols, and the like, including tris(alkoxy)silanol compounds such as tris(tert-butoxy)silanol (TBOS) and tris(tert-pentyloxy)silanol, and bis(tert-alkoxy)silanediol, as well as triethylsilanol. The term "siloxane" refers to of compound having a short repeating unit of silicon and oxygen atoms (either in a chain or a ring) with organic side chains, and includes, without limitation, hexamethylcyclotrisiloxane, octamethylcyclotetrasiloxane, decamethylcyclopentasiloxane, dodecamethylcyclohexasiloxane, hexamethyldisiloxane, octamethyltrisiloxane, decamethyltetrasiloxane and dodecamethylpentasiloxane. The term "aminosilane" refers to a derivative of silane wherein the organic function is a primary or secondary amine and includes, without limitation, APTES (3-aminopropyl)-triethoxysilane, APDEMS (3-aminopropyl)-diethoxy-methylsilane, APDMES (3-aminopropyl)-dimethyl-ethoxysilane and APTMS (3-aminopropyl)-trimethoxysilane. The term "mercaptosilane" includes, without limitation, MPTMS (3-mercaptopropyl)-trimethoxysilane, and MPDMS (3-mercaptopropyl)-methyl-dimethoxysilane. The term "glycidoxysilane" refers to a derivative of silane wherein the organic function is an epoxide and includes, without limitation, GPMES (3-glycidoxypropyl)-dimethyl-ethoxysilane.

In a particular embodiment of the invention, the silica surface of the type I silicon particle of the invention is functionalized by at least one functional group, wherein said functional group is capable of setting up a bond with an enzymatically metabolizable compound, and wherein said at least one functional group is obtained by reacting the silica groups present in the surface of the particle of the invention with aminopropylsilane (APS).

The functional group of the silica surface of the type I silicon particle of the invention is capable of setting up a bond with an enzymatically metabolizable compound. In a particular embodiment, the functional group of the silica surface of the type I particle of the invention is bound to the enzymatically metabolizable compound by a covalent bond. The term "covalent bond" relates to a chemical bond involving the sharing of electron pairs between atoms. In a particular embodiment, the functional group of the silica surface of the particle of the invention is bound to the enzymatically metabolizable compound by a covalent bond. In a particular embodiment, said covalent bond is established by cross-linking via a cross-linking agent.

The term "cross-linking", as used herein, relates to a process of chemically joining two or more molecules by a covalent bond, wherein the cross-linking agent (or cross-linker) comprises reactive ends to specific functional groups. The reactive groups of the cross-linking agent are separated by a spacer chain (also called "spacer arm") of a certain length which determines the distance at which the two residues are crosslinked. Cross-linkers are selected on the basis of their chemical reactivities (i.e., specificity for particular functional groups) and compatibility of the reaction with the application. As the skilled person knows, a suitable cross-linker is selected based on the following characteristics: chemical specificity, spacer arm length, water solubility and cell membrane permeability, same (homobifunctional) or different (heterobifunctional) reactive groups, spontaneously reactive or photoreactive groups, cleavability and the presence of moieties that can be radiolabeled or tagged with another label. Cross-linkers comprise at least two reactive groups, wherein the functional groups that can be targeted for cross-linking include, without limitation, primary amines, sulfhydryls, carbonyls, carbohydrates and carboxylic acids. Cross-linking agents can be divided into groups dependent on the number and similarity of the reactive groups: homobifunctional and heterobifunctional.

The term "homobifunctional cross-linking agent", as used herein, relates to a cross-linking agent comprising two identical reactive ends. The term "heterobifunctional cross-linking agent", as used herein, relates to a cross-linking agent comprising two different reactive ends.

Homobifunctional cross-linkers are used in one-step reactions while the heterobifunctional cross-linkers are used in two-step sequential reactions, where the least labile reactive end is reacted first. Homobifunctional cross-linking agents have the tendency to result in self-conjugation, polymerization, and intracellular cross-linking. On the other hand, heterobifunctional agents allow more controlled two step reactions, which minimizes undesirable intramolecular cross reaction and polymerization.

Homobifunctional cross-linkers include, without limitation, bis (sulfosuccinimidyl) suberate (BSSS, BS3), disuccinimidyl glutarate (DSG), ethylene glicolbis (sulfosuccinimidilsuccinato) (sulfo-EGS), disuccinimidyl suberate (DSS), dithiobis (succinimidyl propionate) (DTSP, Lomant reagent), ethylene glicolbis (succinimidylsuccinate) (EGS), bis (sulfosuccinimidyl) glutarate (BS2G), 3,3 '-dithiobis (sulfosuccinimidylpropionate) (DTSSP), disuccinimidyl tartrate (DST), (bis (2 - (succinimidooxycarbonyloxy] ethyl) sulfone (BSOCOES), 1 ,4-di-(3'-(2'piridilditio)-propionamido) butane (DPDPB), sulfodisuccinimidil tartrate (sulfo DST), dithiobis (succinimidyl propionate) (DSP), ethylene glycol bis (succinimidyl succinate) (EGS).

Heterobifunctional cross-linkers include, without limitation, succinimidyl -4-[N-maleimidomethyl ]cyclohexan-1-carboxylate (SMCC), SANPAH, N-sulfosuccinimidyl -6-[4'-azido-2'-nitrophenylamino]hexanoate(sulfo-SANPAH), m-maleimidobenzoyl-N-hidroxisuccinimide ester (MBS), m-maleimidobenzoyl-N-hidroxisulfosuccinimide ester (sulfo-MBS), N-γ-maleimidobutiriloxisuccinimide ester (GMBS), N-γ-maleimidobutiriloxisulfosuccinimide ester (sulfo-GMBS), N-(ε-maleimidocaproic azido)hydrazide (EMCH), N-(ε-maleimidocaproyloxy)succinimide ester (EMCS), N-(ε-maleimidocaproyloxy) sulfo succinimide ester (sulfo-EMCS), N-(p-maleimidophenyl)isocyanate (PMPI), N-succinimidyl(4-iodoacetyl)aminobenzoate (SIAB), succinimidyl 3-(2-pyridyldithio) propionate (SPDP), succinimidyl6-[3(2-pyridyldithio)propionamido]hexanoate (LC-SPDP), N-succinimidyl bromoacetate (SBA), N-[e-maleimidocaproyloxy] succinimide ester (EMCS), succinimidyl-6-[beta-maleimidopropionamido]hexanoate (SMPH), sulfosuccinimidyl 6-(3'-[2-pyridyldithio]-propionamido)hexanoate (sulfo-LC-SPDP), N-succinimidyl 4-[4-maleimidophenil] butyrate (CMR) , (3-[2-pyridyldithio]propionylhydrazide) (PDPH), N-succinimidyl iodoacetate (SIA), N-(ß-maleimidopropiloxi) succinimide ester (BMPS) and N-5-azido-2-nitrobenzoiloxisuccinimide (ANB-NOS).

When functional groups bound by the cross-linker are considered, cross-linkers include, without limitation, amine to amine cross-linkers, amine to sulfhydryl cross-linkers, carboxyl to amine cross-linkers, photoreactive cross-linkers, sulfhydryl to carbohydrate cross-linkers, sulfhydril to hydroxyl cross-linkers, and sulfhydryl to sulfhydryl cross-linkers.

Amino to amino cross-linkers include, without limitation:
- imidioster crosslinkers including dimethyl adipimidate 2 HCl (DMA), dimethyl pimelimidate 2 HCl (DMP), dimethyl suberimidate 2 HCl (DMS) and dimethyl 3,3'-ditiobispropionimidate 2 HCl (DTBP),
- NHS-ester crosslinkers including bis(succinimidyl)penta(ethyleneglycol) (BS(PEG)5), bis(succinimidyl) nona(ethyleneglycol) (BS(PEG)9), bis(sulfosuccinimidyl) suberate (BS3), bis[2-(succinimidooxycarbonyloxy)etil]sulfone (BSOCOES), disuccinimidyl glutarate (DSG), ditiobis(succinimidyl) propionate (DSP) (Lomants reagent), disuccinimidyl suberate (DSS), disuccinimidyl tartrate (DST), 3,3'-ditiobis(sulfosuccinimidylpropionate) (DTSSP), ethyleneglycol bis(succinimidylsuccinate) (EGS), ethyleneglycol bis(sulfosuccinimidylsuccinate) (sulfo-EGS), Tris(succinimidyl) aminotriacetate (TSAT), and
- other amine-reactive crosslinkers including 1,5-difluoro-2,4-dinitrobenzene (DFDNB)

Amino to sulfhydryl cross-linkers include, without limitation:
- NHS-haloacetyl agents, based on NHS ester and iodoacetyl, bromoacetyl or other haloacetyl reactive groups, and including sulfosuccinimidyl (4-iodoacetyl)aminobenzoate (sulfo-SIAB), succinimidyl(4-iodoacetyl)aminobenzoate (SIAB), succinimidyl 3-(bromoacetamide)propionate (SBAP) y succinimidyl iodoacetate (SIA).
- NHS-maleimide agents, based on N-hydroxysuccinimide and maleimide reactive groups, including sulfosuccinimidyl 4-(N-maleimidomethyl)cyclohexane-1-carboxylate (sulfo-SMCC), NHS-ester and maleimide groups at ends of polyethylene glycol spacer arms (SM(PEG)n series), succinimidyl 4-(N-maleimidomethyl)ciclohexane-1-carboxylate (SMCC), succinimidyl 4-(N-maleimidomethyl)ciclohexane-1-carboxy-(6-amidocaproate) (LC-SMCC), sulfo-EMCS, EMCS, sulfo-GMBS, N-gamma-maleimidobutiryl-oxisuccinimide ester (GMBS), N-kappa-maleimidoundecanoiloxy-sulfosuccinimide ester (sulfo-KMUS), sulfo-MBS, m-maleimidobenzoil-N-hidroxisuccinimida ester (MBS), sulfosuccinimidil 4-(p-maleimidophenyl)butirate (sulfo-SMPB), SMPB, N-alpha-maleimidoacetoxisuccinimide ester (AMAS), BMPS y succinimidyl 6-[(beta-maleimidopropionamide)hexanoate (SMPH).
- NHS-pyridyldithiol agents, based on N-hydroxysuccinimide and pyridyldithiol reactive groups, including 2- pyridyldithiol - tetraoxaoctatriacontane-N-hidroxisuccinimide (PEG12-SPDP), 2-pyridyldithio -tetraoxatetradecane-N-hidroxisuccinimide (PEG4-SPDP), sulfosuccinimidyl 6-[3'-(2- pyridyldithio)propionamide] hexanoate (sulfo-LC-SPDP), succinimidyl 3-(2- pyridyldithio)propionate (SPDP), succinimidyl 6-[3(2- pyridyldithio) propionamide] hexanoate (LC-SPDP), sulfosuccinimidel-6-[alpha-methyl-alpha-(2- pyridyldithio)toluamido] hexanoato (sulfo-LC-SMPT) y 4-succinimidyloxycarbonil-alfa-metil-alpha(2- pyridyldithio)toluen4 (SMPT).

Carboxyl to amine agents include, without limitation, dicyclohexylcarbodiimide (DCC), 1-ethyl-3-[3-dimethylaminopropyl]carbodiimide HCl (EDC o EDAC), N-hydroxysuccinimide (NHS) and N-hydroxysulfoccinimide (S-NHS).

Photoreactive agents include, without limitation, N-5-azide-2-nitrobenzoyloxysuccinimide (ANB-NOS), NHS-diazirine (SDA), sulfo-NHS-diazirine (sulfo-SDA), NHS-LC-diazirine (LC-SDA), sulfo-HHS-LC-diazirine (sulfo-LC-SDA), NHS-SS-diazirine (SDAD), sulfo-NHS-SS-diazirine (sulfo-SDAD), N-sulfosuccinimidyl-6-(4'-azide-2'-nitrophenylamine)hexanoate (sulfo-SANPAH), succinimidyl-[4-(psoralen-8-iloxi)]-butirate (SPB).

Sulfhydryl to carbohydrate agents include, without limitation, N-beta-maleimidopropionic acid hydrazide-TFA (BMPH), N-epsilon-maleimidocaproic acid hydrazide-TFA (EMCH), N-kappa-maleimidoundecanoic acid hydrazide-TFA (KMUH), 4-(4-N-maleimidophenyl)butyric acid hydrazide-HCl; (MPBH) and 3-(2-pyridyldithio)propionyl hydrazide (PDPH).

Sulfhydryl to hydroxyl agents include, without limitation, N-(p-maleimidophenil)isocyanate (BMPI).

Sulfhydryl to sulfhydryl agents include, without limitation, 1,8-bismaleimidodiethyleneglycol (BM(PEG)2), 1,1-bismaleimidotriethyleneglicol (BM(PEG)3), 1,4-bismaleimidobutane (BMB), 1,4 bismaleimidil-2,3-dihidroxibutane (BMDB), bismaleimidohexane (BMH), bismaleimidoetane (BMOE).

In a particular embodiment of the present invention, molecules containing reactive groups capable of being conjugated by cross-linkers are proteins or peptides. For protein crosslinking, protein functional groups for cross-linkers comprise amine groups, epsilon-amine groups of lysines, terminal alpha-amino groups, cysteines sulfhydryl groups (-SH or thiol groups), carbohydrate groups (in the case of glycoproteins) or carboxyl groups.

Protein cross-linkers for amine groups, epsilon-amine groups of lysines, and terminal alpha-amino groups include, without limitation, imidoesters and NH-hydroxysuccinimide esters (NHS-esters).

Protein cross-linkers for sulfhydryl groups include, but are not limited to, maleimides, haloacetyls (such as iodoacetyl) and pyridyl disulfide (pyridyldithioles).

Protein cross-linkers for carbonyl groups (such as aldehydes or ketones) by oxidative treatment of glycoproteins carbohydrates include, without limitation, reagents comprising hydrazides (-NH-NH₂-).

Protein cross-linkers for carboxyl groups include, without limitation, carbodiimides.

### 2.1.3 Enzimatically metabolizable compound

The silicon type I particle of the invention further comprises an enzimatically metabolizable compound. The enzimatically metabolizable compound that can be used within the context of the present invention can be any compound that (i) can be metabolized by a cellular enzyme and (ii) can be linked both to the silica surface of the silicon particle through a functional group and to a targeting moiety.

Compounds that can be metabolized by a cellular enzyme include all natural and synthetic substrates of cellular enzymes and include, without limitation, carbohydrates, lipids, peptides, proteins, and nucleic acids.

A non-exhaustive list of enzimatically metabolizable compounds that can be used within the context of the present invention is shown in "Enzyme Explorer" by Sigma-Aldrich (http://www.sigmaaldrich.com/life-science/metabolomics/enzyme-explorer/substrate-index.html).

In a particular embodiment, said enzimatically metabolizable compound is linked to the silica surface of the particle of the invention by a covalent bond. In a particular embodiment, the enzimatically metabolizable compound is linked to the targeting moiety by a covalent bond as well. In an alternative particular embodiment, the enzimatically metabolizable compound of the particle of the invention is linked to the targeting moiety by a weak interaction. The term "weak interaction", as used herein, refers to hydrophylic or hydrophobic interactions, electrostatic interactions or Van der Waals interactions.

In a particular embodiment, said enzimatically metabolizable compound is a carbohydrate. In a more particular embodiment, said enzimatically metabolizable compound is a carbohydrate selected from the group consisting of mono-, di- or polysaccharides degradable by mammalian cells. In a preferred embodiment, the enzimatically metabolizable compound is a carbohydrate, such as pyranoside, preferably a glucopyranoside, for example a glycoside of glucopyranose. In a specific embodiment, the enzimatically metabolizable compound is 2-acetamido-2-deoxy-β-d-glucopyranosyloxyacetic acid.

The enzymatically metabolizable compound is susceptible of being metabolized by the suitable enzyme into the target cell. In a particular embodiment, the enzymatically metabolizable compound is a compound susceptible of being degraded into smaller fragments by a catabolic enzyme of a target cell, wherein said catabolitic enzyme includes, without limitation, a nuclease (enzyme capable of cleaving the phosphodiester bonds between the nucleotide subunits of nucleic acids), a phosphodiesterase (enzyme that breaks a phosphodiester bond), a lipase (enzyme that catalyzes the hydrolysis lipids), a phosphatase (enzyme that removes a phosphate group from its substrate by hydrolysing phosphoric acid monoesters into a phosphate ion and a molecule with a free hydroxyl group), a glycoside hydrolase (or glycosyl hydrolase, enzyme involved in the hydrolysis of glycosidic bonds in complex carbohydrates), a protease (enzyme for proteolysis or protein catabolism by hydrolysis of the peptide bonds that link amino acids together in the polypeptide chain forming the protein) and a peptidase (enzyme for proteolysis or protein catabolism by hydrolysis of the peptide bonds that link amino acids together in the polypeptide chain forming the peptide).

In another particular embodiment, the enzymatically metabolizable compound is a compound susceptible of being degraded into smaller fragments by a catabolic enzyme of a target cell, wherein said catabolic enzyme is a lysosomal enzyme including, without limitation, a lysosomal protease, a lysosomal acid lipase, a lysosomal nuclease and a lysosomal glycoside hydrolase, preferably a lysosomal glycoside hydrolase, more preferably a lysosomal glucohydrolase.

### 2.1.4 Targeting moiety

The silicon type I particle of the invention further comprises a targeting moiety, wherein said targeting moiety is linked to the particle through the enzymatically metabolizable compound.

In a particular embodiment, said targeting moiety is selected from the group comprising an antibody or an antigen binding fragment thereof, a binding structure, an aptamer, a peptide or a small molecule. As the skilled person understands, the election of the particular targeting moiety of the particle is based on the particular cell to which the particle is to be targeted.

In a particular embodiment, the targeting moiety is an antibody or an antigen binding fragment thereof. The antibody of the targeting moiety includes any immunological agent capable of binding to a ligand with high affinity, including IgG, IgM, IgA, IgD and IgE, as well as molecules similar to antibodies which have an antigen binding site, such as Fab', Fab, F(ab')2, single domain antibodies or DABS, Fv, scFv and the like.

Various techniques have been developed for the production of antibody fragments. Traditionally, these fragments are derived via proteolytic digestion of intact antibodies but more recently these fragments can be produced directly by recombinant host cells. Papain digestion of antibodies produces two identical antigen-binding fragments, called "Fab" fragments, each with a single antigen-binding site, and a residual "Fc" fragment, which name reflects its ability to crystallize readily. Pepsin treatment yields an F(ab')₂ fragment that has two antigen-binding sites and is still capable of cross-linking antigen. "Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind the antigen, although with lower affinity than the entire binding site. The Fab fragment also contains the constant domain of the light chain and the first constant domain (CHI) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')Z antibody fragments originally were produced as pairs of Fab' fragments which have hinge cysteines between them. Other chemical couplings of antibody fragments are also known. "Single-chain Fv" or "scFv" antibody fragments comprise the V_{H} and V_{L} domains of an antibody, wherein these domains are present in a single polypeptide chain. Preferably, the Fv polypeptide further comprises a polypeptide linker between the VH and VL domains which enables the scFv to form the desired structure for antigen binding. For a review of scFv see Pluckthun in The Pharmacology of Monoclonal Antibodies, vol. 113, Rosenburg and Moore eds., Springer-Verlag, N.Y., pp. 269-315 (1994).

The targeting moiety of the silicon type I particle of the invention is able to specifically recognize and bind to a molecule located in the surface of a target cell, so the silicon particle of the invention can be targeted to a target cell, such as a tumoral cell, by means of the targeting moiety. According to the invention, molecules exposed in the surface of the target cell and able to be specifically recognized and/or bound by a targeting moiety of the particle are selected from the group comprising a cell receptor, a cell adhesion molecule, an integrin, a surface glycoprotein, and a co-stimulatory molecule. In a particular embodiment, the targeting moiety of the silicon type I particle of the invention is an antibody or an antigen binding fragment thereof, wherein said antibody or fragment thereof specifically recognizes and binds to a cell receptor located in the surface of a target cell. In a more particular embodiment, the targeting moiety of the silicon type I particle of the invention is an antibody or an antigen binding fragment thereof, wherein said antibody or fragment thereof specifically recognizes and binds to a cell receptor located in the surface of a target cell, wherein said cell receptor is overexpressed in the surface of a tumoral cell with respect to a non-tumoral cell.

Cell surface receptors include, without limitation, ion-channel-linked receptors, G-protein-linked receptors, and enzyme-linked receptors. Non-limiting examples of surface cell receptors, encoded by genes described to contain mutations linked to tumor formation, include the following: ALK (anaplastic lymphoma kinase (Ki-1), Q9UM73), BMPR1A (bone morphogenetic protein receptor, type IA, P36894), CD47 receptor (Q08722), CD76 receptor, EGFR (epidermal growth factor receptor, v-erb-b, P00533), ERBB2 (v-erb-b2, erythroblastic leukemia viral, P04626), FGFR1 (fibroblast growth factor receptor 1, P11362), FGFR2 (fibroblast growth factor receptor 2, P21802), FGFR3 (fibroblast growth factor receptor 3, P22607), FLT3 (fms-related tyrosine kinase, P36888), FLT4/VEGFR3/VPF (fms-related tyrosine kinase/vascular endothelin growth factor/vascular permeability factor receptor, P35916), GLUT1-12 (solute carrier family 2, facilitated glucose transporter members 1-13; P11166, P11168, P11169, P14672, P22732, Q9UGQ3, Q6PXP3, Q9NY64, Q9NRM0, 095528, Q9BYW1, and Q8TD20, respectively), IL21R (interleukin 21 receptor, Q9HBE5), IRTA1 (immunoglobulin superfamily receptor translocation associated 1, NP_112572), c-KIT (v-kit Hardy-Zuckerman 4 feline sarcoma viral oncogene homolog)/stem-cell receptor (P10721), MET (met proto-oncogene)/hepatocyte growth factor receptor (P08581), MHC I, II, III and non-classical MHC (major histocompatibility complex), NTRK3 (neurotrophic tyrosine kinase, receptor, type 3, Q16288), PDGFRA platelet-derived growth factor, alpha-receptor (P16234), PDGFRB (platelet-derived growth factor receptor, beta polypeptide, NP_002600), RARα (retinoic acid receptor alpha, P10276), RET (ret proto-oncogene, P07949), TEK/TIE2 (P42680), TFRC (transferrin receptor)/p90/CD71, P02786), TNFRSF6 (tumor necrosis factor receptor superfamily, member 6)/FAS, P25445), Toll-like receptors, including without limitation Toll-like receptor 1, Q15399), TSHR (thyroid stimulating hormone receptor, P16473) and VEGFR (P35968) (protein identification numbers according to SwissProt/RefSeq are indicated for each receptor).

The targeting moiety is linked to the silicon type I particle of the invention through the enzymatically metabolizable compound. In a particular embodiment, the targeting moiety is linked to the enzymatically metabolizable compound of the silicon type I particle by weak interactions, as described above. In a particular alternative embodiment, the targeting moiety is linked to the enzymatically metabolizable compound of the silicon particle via a cross-linking agent. Agents suitable for cross-linking have been previously described. In a particular alternative embodiment, the targeting moiety is linked to the enzymatically metabolizable compound of the silicon particle by click chemistry. The term "click chemistry", as used herein, relates to a chemistry tailored to generate substances quickly and reliably by joining small units together, involving a modular protocol for organic synthesis that utilizes powerful, highly reliable and selective reactions for the rapid synthesis of compounds, as described in Kolb HC et al. 2001 Angew Chem Int Edit 40(11): 2044-2021.

### 2.2 Silicon type II particles

### 2.2.1 Silicon body (core)

As previously described for the type I silicon particle of the invention, the type II silicon particle of the invention comprises a silicon body (core).

In a particular embodiment, the silicon body of the type II particle of the invention comprises a solid silicon body.

In another particular embodiment, the silicon body of the type II particle of the invention does not comprise any pores. In another embodiment, the silicon body of the type II particle of the invention comprises one or more pores. The size of the pores of the silicon body eventually present in the particle of the invention is included within the nanomolar range, preferably about 1-2 nm. Suitable methods to determine pore size, as well as methods for obtaining silicon particles, have been previously described in relation to type I particles of the invention.

### 2.2.2 Silica surface (shell)

The type II silicon particle of the invention further comprises a silica surface (shell), wherein said silica surface is located surrounding the silicon body or core of the particle.

The silica surface of the type II silicon particle of the invention is functionalized by at least one functional group, wherein said functional group is capable of setting up a bond with a targeting moiety. Suitable functional groups for use within the context of the invention have been previously described in relation to type I particles of the invention and include, without limitation, halosilanes, organosilanes, silanoles, siloxanes, aminosilanes, mercaptosilanes and glycidoxysilanes.

The functional group of the silica surface of the type II silicon particle of the invention is capable of setting up a bond with a targeting moiety. In a particular embodiment, the functional group of the silica surface of the type II particle of the invention is bound to a targeting moiety by a covalent bond. In a more particular embodiment, said covalent bond is established by cross-linking via a cross-linking agent. Suitable cross-linkers according to the invention have been described previously in relation to type I silicon particles.

### 2.2.3 Targeting moiety

The silicon type II particle of the invention further comprises a targeting moiety, wherein said targeting moiety is directly linked to the functionalized silica surface of the particle. Thus, regarding the silicon type II particle of the invention, the targeting moiety both targets the silicon particle to a particular target cell and protects the silica surface of the particle from dissolution until its metabolization.

The targeting moiety of the silicon type II particle of the invention is selected from the group comprising an antibody or an antigen binding fragment thereof, a binding structure, an aptamer, a peptide or a small molecule, as previously described in relation to the silicon type I particle of the invention. As the skilled person understands, the election of the particular targeting moiety of the particle is based on the particular cell to which the particle is to be targeted. In a particular embodiment, is an antibody or an antigen binding fragment thereof.

The targeting moiety of the silicon type II particle of the invention is able to specifically recognize and bind to a molecule located in the surface of a target cell, so the silicon particle of the invention can be targeted to a target cell, such as a tumoral cell, by means of the targeting moiety. According to the invention, molecules exposed in the surface of the target cell and able to be specifically recognized and/or bound by a targeting moiety of the particle are selected from the group comprising a cell receptor, a cell adhesion molecule, an integrin, a surface glycoprotein, and a co-stimulatory molecule. In a particular embodiment, the targeting moiety of the silicon type II particle of the invention is an antibody or an antigen binding fragment thereof, wherein said antibody or fragment thereof specifically recognizes and binds to a cell receptor located in the surface of a target cell. In a more particular embodiment, the targeting moiety of the silicon type II particle of the invention is an antibody or an antigen binding fragment thereof, wherein said antibody or fragment thereof specifically recognizes and binds to a cell receptor located in the surface of a target cell, wherein said cell receptor is overexpressed in the surface of a tumoral cell with respect to a non-tumoral cell.

Cell surface receptors have been previously described in the context of the silicon type II particle of the invention.

The targeting moiety is linked to the silicon type II particle of the invention through the functional group than functionalizes the silica surface surrounding the silicon body. In a particular embodiment, the targeting moiety is linked to the functionalized silica surface of the silicon type II particle by weak interactions, as described above in the context of the silicon type I particle. In a particular alternative embodiment, the targeting moiety is linked to the functionalized silica surface of the silicon type II particle via a cross-linking agent. Agents suitable for cross-linking have been previously described in the context of the silicon type I particle. In a particular alternative embodiment, the targeting moiety is linked to the functionalized silica surface of the silicon type II particle by click chemistry.

### 3. Process for producing the particles of the invention

In a further aspect, the invention relates to a process for producing a particle of the invention, hereinafter referred to as the "process of the invention", selected from the group consisting of "Process I" and "Process II",
- wherein Process I comprises:
   i) contacting a silicon particle comprising a silicon body and a functionalized silica surface with an enzymatically metabolizable compound under conditions suitable for binding the compound to the functionalized silica surface; and
   ii) contacting the product resulting from step i) with a targeting moiety under conditions suitable for binding the targeting moiety to the compound bound to the functionalized silica surface, and
- wherein Process II comprises contacting a silicon particle comprising a silicon body and a functionalized silica surface with a targeting moiety under conditions suitable for binding the targeting moiety to the functionalized silica surface.

According to Process I, a silicon type I particle is obtained. Under step i) of Process I, a silicon particle comprising a silicon body and a functionalized silica surface is contacted with an enzymatically metabolizable compound under conditions suitable for binding the compound to the functionalized silica surface.

Methods for obtaining a silicon particle comprising a silicon body and a silica surface are known by the skilled person and include, without limitation, the methods described in WO2012101306, Harris JT et al. cited *supra* and Shi L *et al.* cited *supra.* In a particular embodiment, small silicon particles (smaller than 400 nm) are produced by mechanical milling of silicon powder together with a further particle separation through a colloidal sedimentation process. Larger a-Si:H particles (380-650 nm) are prepared by trisilane (Si₃H₈; 100%) decomposition in supercritical n-hexane (95%, anhydrous) in a titanium reactor with the internal volume of 10 mL. The a-Si:H particle size is adjusted by changing the amount of hexane and trisilane used in the reaction. In a particular embodiment, a-Si:H particles with 380 nm diameter and 50-60 at.% H-content are made by loading 6.4 mL of n-hexane and 10 µL of trisilane into a cylindrical titanium reactor with 10 mL internal volume in a nitrogen-filled glovebox. The reactor is sealed and removed from the glovebox. A brass heating block is preheated to 50°C above the desired reaction temperature. For instance, for a reaction run at 420°C, the heating block is preheated to 470°C. After placing the reactor into the preheated block, the temperature typically drops by about 40°C below the desired reaction temperature and returns to the reaction temperature in 3 min. After 10 min of total heating time, the reactor is removed from the heating block and submerged in an ice bath. After cooling to room temperature, the reactor is opened to collect the product with 5 mL of chloroform. The product is centrifuged at 8000 rpm for 5 min to precipitate the particles. The supernatant is discarded. The particles are dispersed in 2 mL of chloroform and stored in air. A second step concerns submitting particles to a vacuum annealing treatment to obtain high refractive index SCs. X-ray diffraction and Raman spectroscopy showed that the as-synthesized colloids are made of hydrogenated amorphous silicon. Then monodisperse particles are submitted to an annealing process at 500°C for removing hydrogen, thus increasing the refractive index of particles up to a value equivalent to the 90% of the refractive index of bulk silicon.

In another particular embodiment, porous silicon particles are obtained by a process based on the decomposition of disilane gas (Si₂H₆) by means of chemical vapor deposition (CVD). It is similar to the synthesis process of silicon colloids, where the gas is introduced in a reactor whose walls are heated at high temperatures for a certain time, usually higher than 400°C. During this process, SiₙHₘ clusters grow in the gas phase and they become highly spherical particles thanks to surface tension forces. At the same time, there is a hydrogen desorption process from the clusters that makes the hydrogen content decrease progressively until they become hydrogenated amorphous silicon (a:Si-H) colloids. In order to obtain porous silicon particles, the heating process is stopped at an early stage, before the formation of amorphous silicon colloids has finished. In this way, porous particles with an undetermined composition of silicon and hydrogen atoms are obtained. In a more particular embodiment, porous silicon particles were synthesized as described in the section entitled "Examples" below.

The silica surface surrounding the silicon body (generated, for example, by contacting the silicon body with air) is functionalized by contacting the silica groups present in the silica surface with a suitable functionalizing agent such as, for example, a silane, e.g., an halosilane, an organosilane, a silanol, a siloxane, an aminosilane, a mercaptosilane, a glycidoxysilane, etc., such as those mentioned in section "2.2 Particle silica surface (shell)". In a particular embodiment, the silica surface of the silicon particle is functionalized with aminopropylsilane (APS). The reaction of the silica groups present in the silica surface with the functionalizing agent is carried out under conditions suitable for functionalizing the silica surface; said conditions refer to particular pH, medium composition, agents concentration, temperature, etc. that allow the reaction of silica groups with the functionalizing agent and functionalization of the silica surface. Said conditions depend, among other features, on the nature of the functionalizing agent; the skilled person in the art can select the most suitable conditions for performing said reaction.

The expression "conditions suitable for the binding of an enzymatically metabolizable compound to a functionalized silica surface", as used herein, refers to particular pH, medium composition, agents concentration, temperature, etc. that allow the reaction of the enzimatically metabolizable compound and the functional groups of the functionalized silica surface and binding of said enzimatically metabolizable compound to said functionalized silica surface. Said conditions depend, among other features, on the nature of the functional groups present in the functionalized silica surface and the enzimatically metabolizable compound to be bound; the skilled person in the art can select the most suitable conditions for performing said reaction.

In step ii) of Process I, the product resulting from step i) is contacted with a targeting moiety under conditions suitable for binding the targeting moiety to the compound bound to the functionalized silica surface.

The expression "conditions suitable for the binding of a targeting moiety to an enzymatically metabolizable compound bound to the functionalized silica surface" as used herein refers to particular pH, medium composition, agents concentration, temperature, etc. that allow the binding of the targeting moiety to the enzimatically metabolizable compound previously bound to the functionalized silica surface of the particle of the invention; said conditions are known, or can be determined by the skilled person in the art, for a particular targeting moiety and a particular enzimatically metabolizable compound by conventional methods. Said conditions depend, among other features, on the nature of the targeting moiety, the enzimatically metabolizable compound and cross-linker used (if necessary); the skilled person in the art can select the most suitable conditions for performing said reaction.

According to Process II, a silicon type II particle is obtained by contacting a silicon particle comprising a silicon body and a functionalized silica surface with a targeting moiety under conditions suitable for binding the targeting moiety to the functionalized silica surface.

Methods for obtaining a silicon particle comprising a silicon body and a silica functionalized surface have been previously described in connection with Process I.

The expression "conditions suitable for the binding of a targeting moiety to a functionalized silica surface", as used herein, refers to particular pH, medium composition, agents concentration, temperature, etc. that allow the reaction of the targeting moiety and the functional groups of the functionalized silica surface and binding of said targeting moiety to said functionalized silica surface. Said conditions depend, among other features, on the nature of the functional groups present in the functionalized silica surface and the targeting moiety to be bound; the skilled person in the art can select the most suitable conditions for performing said reaction.

### 4. Compositions of the invention

In a further aspect, the present invention relates to a composition, hereinafter referred to as the "composition of the invention", comprising at least a particle of the invention. In a particular embodiment, the composition of the invention further comprises a suitable medium, wherein the particles of the invention are substantially stable, i.e. aqueous solution and physiologically compatible solutions.

In a further aspect, the invention relates to a pharmaceutical composition, hereinafter referred to as the "pharmaceutical composition of the invention", comprising a therapeutically effective amount of the particles of the invention together with a pharmaceutically acceptable excipient.

The combination of the particles of the invention and the pharmaceutically acceptable excipient may be found in an isolated dosage form or in combination with additional active agents. Excipients will be selected in view of the elected pharmaceutical dosage form; illustrative, non-limitative, examples of said excipients include sugars, starches, celluloses, gums, proteins, phosphate buffered saline solutions, water, emulsions, such as oil/water emulsions, humectants, sterile solutions, etc.

In a particular embodiment, the pharmaceutical composition of the invention will be formulated as a solid pharmaceutical dosage form (e.g., tablets, capsules, coated tablets, granules, sterile solids that can be reconstituted to provide liquid forms, etc.), or as a liquid dosage form (e.g., suspensions, emulsions, etc.), or even as a semisolid dosage form (e.g., gels, pomades, creams and the like). The pharmaceutical composition of the invention may be administered by any suitable route, including, without limitation, oral, parenteral (e.g., intradermal, intravenous, intramuscular, intraarterial, intramedullary, intrathecal, intraventricular, subcutanous, intraperitoneal, etc.), topical, etc. A review of the different dosage forms of active agents and excipients and their manufacturing processes is provided in "Tratado de Farmacia Galénica", C. Faulí and Trillo, Luzán 5, S.A. de Ediciones, 1993 and in "Remington's Pharmaceutical Sciences" (A.R. Gennaro, Ed.), 20th edition, Williams & Wilkins PA, USA (2000). Intravenous or oral administrations are preferred for a curative effect.

The pharmaceutical composition of the invention may further comprise additional microbicidal agents directed against a wide variety of bacteria, viruses, fungi, etc. The pharmaceutical composition of the invention may be combined with other active ingredients, such as an anticancer agent, an immunomodulator, etc.

The person skilled in the art will appreciate that the nature of the excipient in the pharmaceutical composition of the invention will depend to a great extent on the administration route.

In the case of the compositions formulated for their oral use, the pharmaceutical composition of the invention normally will contain the particles of the invention mixed with one or more pharmaceutically acceptable excipients. These excipients can be, for example, inert fillers or diluents, such as sucrose, sorbitol, sugar, mannitol, microcrystalline cellulose, starches, including potato starch, calcium carbonate, sodium chloride, lactose, calcium phosphate, calcium sulfate or sodium phosphate; crumbling agents and disintegrants, for example cellulose derivatives, including microcrystalline cellulose, starches, including potato starch, sodium croscarmellose, alginates or alginic acid and chitosans; binding agents, for example sucrose, glucose. sorbitol, acacia, alginic acid, sodium alginate, gelatin, starch, pregelatinized starch, microcrystalline cellulose, aluminum magnesium silicate, sodium carboxymethylcellulose, methylcellulose, hydroxypropyl methylcellulose, ethylcellulose, polyvinylpyrrolidone, polyvinyl acetate or polyethylene glycol, and chitosans; lubricating agents, including glidants and antiadhesive agents, for example magnesium stearate, zinc stearate, stearic acid, silicas, hydrogenated vegetable oils or talc.

The pharmaceutically acceptable excipients can include solvents, buffering agents, preservatives, wetting agents, chelating agents, antioxidants, stabilizers, emulsifiers, suspending agents, gel-forming agents, bases for ointments, penetration enhancers, etc.

Solvents include, without limitation, water, alcohols, vegetable or marine oils (for example, edible oils, such as almond oil, castor oil, cocoa butter, coconut oil, maize oil, cotton oil, linseed oil, olive oil, palm oil, peanut oil, poppy oil, rapeseed oil, sesame oil, soy oil, sunflower oil and tea oil), mineral oils, fatty oils, liquid paraffin, polyethylene glycols, propylene glycols, glycerol, liquid polyalkylsiloxanes and mixtures thereof. Examples of buffering agents are, for example, citric acid, acetic acid, tartaric acid, lactic acid, phosphoric hydrogen acid, diethylamine, etc. Suitable examples of preservatives for use in the compositions are parabens, such as methyl, ethyl or propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, benzalkonium chloride and benzyl alcohol, or mixtures of preservatives. Examples of wetting agents are glycerine, propylene glycol, sorbitol, lactic acid, urea and mixtures thereof Examples of chelating agents are sodium EDTA and citric acid. Examples of antioxidants are butylated hydroxyanisole (BHA), ascorbic acid and derivatives thereof, tocopherol and derivatives thereof, cysteine and mixtures thereof. Examples of emulsifiers are natural gums, for example acacia gum or gum tragacanth; natural phosphatides, for example as soy lecithin; sorbitan monooleate derivatives; wool fats; wool alcohols; sorbitan esters; monoglycerides; fatty alcohols; fatty acid esters (for example, fatty acid triglycerides), and mixtures thereof. Examples of suspending agents are, for example, cellulose and cellulose derivative, such as, for example, carboxymethylcellulose, hydroxyethylcellulose, hydroxypropylcellulose, hydroxypropyl methylcellulose, carrageenan, acacia gum, gum arabic, tragacanth and mixtures thereof. Examples of gel bases are viscosity-enhancing agents or components capable of collecting exudates from a wound: liquid paraffin, polyethylene, fatty oils, silica or colloidal aluminum, zinc soaps, glycerol, propylene glycol, tragacanth, carboxyvinyl polymers, magnesium and aluminum silicates, Carbopol®, hydrophilic polymers, such as, for example, starch or cellulose derivatives, such as, for example, carboxymethylcellulose, hydroxyethylcellulose and other cellulose derivatives, water-swellable hydrocolloids, carrageenans, hyaluronates (e.g., hyaluronate gel possibly containing sodium chloride) and alginates, including propylene glycol alginate. Examples of ointment bases are, for example, bee wax, paraffin, cetanol, cetyl palmitate, vegetable oils, sorbitan esters and fatty acids (Span), polyethylene glycols and sorbitan ester and fatty acid and ethylene oxide condensation products, for example polyoxyethylene sorbitan monooleate (Tween). Examples of hydrophobic or water-emulsifying ointment bases are paraffins, vegetable oils, animal fats, synthetic glycerides, waxes, lanoline and liquid polyalkylsiloxanes. Examples of hydrophilic ointment bases are solid macrogols (polyethylene glycols). Other examples of ointment bases are triethanolamine soaps, sulfated fatty alcohol and polysorbates. Examples of other excipients include polymers such as carmellose, carmellose sodium, hydroxypropylmethylcellulose, hydroxyethylcellulose, hydroxypropylcellulose, pectin, xanthan gum, locust bean gum, acacia gum, gelatin, carbomer, emulsifiers such as vitamin E, glyceryl stearates, cetanyl glucoside, collagen, carrageenan, hyaluronates and alginates and chitosans.

The pharmaceutical compositions of the invention can also be presented in the form of suspensions, emulsions or dispersions. Said compositions contain the particles of the invention in mixture with a dispersing or wetting agent, a suspending agent and/or one or more preservatives and other pharmaceutically acceptable excipients. Suitable dispersing or wetting agents are, for example, natural phosphatides, for example lecithin or soy lecithin; the condensation products of ethylene oxide with, for example, a fatty acid, a long chain aliphatic alcohol or a partial ester derived from fatty acids and a hexitol or hexitol anhydride, for example polyoxyethylene stearate, polyoxyethylene sorbitol monooleate, polyoxyethylene sorbitan monooleate, etc. Suitable suspending agents are, for example, natural gums, such as, for example, acacia gum, xanthan gum or tragacanth gum; celluloses, such as, for example, sodium carboxymethylcellulose, microcrystalline cellulose (for example, Avicel® RC 591, methylcellulose); alginates and chitosans, such as, for example, sodium alginate, etc. Suitable examples of preservatives for use in the pharmaceutical composition of the invention are the same as those mentioned above.

Suitable oral pharmaceutical compositions of the invention can be in the form of a particulate formulation or in the form of a solid, semisolid or fluid dosage form, including, for example, granules, granulates, sachets, tablets, capsules, , etc., as well as fluid or liquid formulations, such as, for example, suspensions, emulsions, dispersions and mixtures. Moreover, the pharmaceutical composition of the invention can be in the form of powders, dispersible powders or granules suitable for preparing an aqueous suspension by adding a liquid medium, such as, for example, an aqueous medium.

In those cases in which the pharmaceutical composition of the invention is in the solid unit dosage form (for example, a tablet or a capsule), the unit dosage form can be provided with a coating, for example, with a sugar coating, a film coating (for example, based on hydroxypropylmethylcellulose, methylcellulose, methylhydroxyethylcellulose, hydroxypropylcellulose, carboxymethylcellulose, acrylate copolymers (Eudragit), polyethylene glycols and/or polyvinylpyrrolidone) or an enteric coating (for example, based on methacrylic acid copolymer (Eudragit), cellulose acetate phthalate, hydroxypropylmethylcellulose phthalate, hydroxypropylmethylcellulose acetate succinate, polyvinyl acetate phthalate, lacquer gum and/or ethylcellulose). Moreover, a time delay material such as, for example, glyceryl monostearate or glyceryl distearate, can be used.

The pharmaceutical composition of the invention may be also administered to a subject in need thereof via parenteral or intratumoral. The term "parenteral" as used herein includes, for example, intradermal, intravenous, intramuscular, intraarterial, intramedullary, intrathecal, intraventricular, subcutanous, or intraperitoneal administration.

In addition, the pharmaceutical composition of the invention may suitably be administered by pulse infusion, e.g. with declining doses of the particles of the invention. Preferably the dosing is given by injections, most preferably intravenous or subcutaneous injections, depending in part on whether the administration is brief or chronic.

In a particular embodiment, the pharmaceutical composition of the invention may be adapted for parenteral administration, such as sterile solutions, suspensions or lyophilized products in the appropriate unit dosage form. Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, CremophorEM (BASF, Parsippany, N.J.) or phosphate buffered saline (PBS). In all cases, the composition must be sterile and should be fluid to the extent to allow easy syringability. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and/or gelatin.

Sterile injectable solutions can be prepared by incorporating the active compound (e.g., the particle of the invention) in the required amount in an appropriate solvent with one or a combination of ingredients enumerated above, as required, followed by filtered sterilization. Generally, dispersions are prepared by incorporating the particle of the invention into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

In a particular embodiment, said pharmaceutical composition is administered via intravenous or intratumoural. Adequate excipients can be used, such as bulking agents, buffering agents or surfactants. The mentioned formulations will be prepared using standard methods such as those described or referred to in the Spanish and US Pharmacopoeias and similar reference texts.

It is especially advantageous to formulate the pharmaceutical composition of the invention, namely, oral or parenteral compositions, in dosage unit forms for ease administration and uniformity of dosage. Dosage unit form as used herein refers to physically discrete units suited as unitary dosages for the subject to be treated; each unit containing a predetermined quantity of active compound (particle of the invention) calculated to produce the desired therapeutic effect in association with the required pharmaceutical carrier. The specification for the dosage unit forms of the invention are dictated by and directly dependent on the unique characteristics of the particle of the invention and the particular therapeutic effect to be achieved, and the limitations inherent in the art of compounding such an active compound for the treatment of subjects.

The particles of the invention (active agents) will typically be administered once or more times a day for example 1, 2, 3 or 4 times daily, with typical total daily doses in the range of from 0.001 to 1,000 mg/kg body weight/day, preferably about 0.01 to about 100 mg/kg body weight/day, most preferably from about 0.05 to 10 mg/kg body weight/day. The pharmaceutical composition will be formulated in order to contain the desired amount, such as a therapeutically effective amount of the particle of the invention.

The amount of the pharmaceutical composition of the invention to be applied depends, among other features, on the concentration of the particles of the invention. The concentration of the particles of the invention in the pharmaceutical composition of the invention depends on the concentration of the particles of the invention, on the severity of the disease which is to be prevented or treated and on the age and condition of the subject to be treated (patient). The methods applied for the selection of relevant concentrations of the particles of the invention in the pharmaceutical composition of the invention are well known for a person skilled in the art and can be carried out according to guidelines for Good Clinical Practice (GCP) or Investigational New Drug (IND) Exemption regulations, according to what is described, for example, in the International Standard ISO/DIS 14155 for clinical investigation of medical devices, 1994, and "ICH" (International Conference on Harmonization): harmonized tripartite guideline for Good Clinical Practice, Brookwood Medical Publications, Ltd., Surrey, UK, 1996. A person skilled in the art will be able to, by means of the use of the methods described in standard textbooks, of the guidelines and of the regulations described above, as well as by means of the use of general common knowledge within this field, select the exact dosage regimen to be performed for the particles of the invention and/or select other active substances and dosage forms using only routine experimenting processes.

The pharmaceutical composition of the invention can be prepared in a way known by itself and familiar to persons with experience in the art.

The pharmaceutical compositions of the invention can be included in a container, pack, or dispenser together with instructions for administration.

The particles of the invention as well as the pharmaceutical composition of the invention may be used with other drugs to provide a combination therapy, such as a combination therapy for treating cancer. The other drugs may form part of the same pharmaceutical composition, or can be provided as a separate composition for administration at the same time or at different time. Thus, in a particular embodiment, said additional drug is an anti-cancer agent.

The term "anti-cancer agent" or "anti-tumoral" agent" or "antineoplastic agent", as used herein, refers to an agent that is useful in the treatment of cancer. Anti-cancer agents include radionuclides and drugs useful for chemotherapy.

In the context of the radionuclides useful for radiotherapy, alpha-emitting, beta-emitting and gamma-emitting radionuclides are particularly useful. Agents suitable for use in radiotherapy are well-known by the skilled person. Illustrative examples that are useful in the context of the present invention include, without limitation, alpha emitters, such as ²¹³Bi and ²¹¹At; beta emitters, such as ⁹⁰Y, ^{99m}Tc, ¹⁷⁷Lu, and ⁶⁷Cu; and gamma-emitters, such as ¹³¹I.

In the context of the drugs useful for chemotherapy, agents suitable for use in chemotherapy are well-known by the skilled person. As used herein, the term "drug" refers to a chemical substance used in the treatment, cure, or prevention of a disease or condition, e.g., cancer, etc. The chemical nature of the drug can vary broadly, e.g. it can be a small molecule, a peptide, and so on. A preferred class of drugs are those that intervene at the nuclear level in the cell. Although different and numerous kinds of drugs can be used within the context of the invention, in a particular embodiment, the present invention contemplates that the drug is selected from the group consisting of an alkylating agent, an antimetabolite, a topoisomerase inhibitor and an anthracycline.

As used herein, the term "alkylating agent" or "alkylating antineoplastic agent" refers to an agent that mediates the transfer of an alkyl group from one molecule to DNA. The alkyl group may be transferred as an alkyl carbocation, a free radical, a carbanion or a carbene (or their equivalents). Alkylating agents are used in chemotherapy to damage the DNA of cancer cells. The alkylating agents are generally separated into six classes:
- nitrogen mustards, such as mechlorethamine, cyclophosphamide, ifosfamide, melphalan, chlorambucil, etc.;
- ethylenamine and methylenamine derivatives, including altretamine, thiotepa and the like;
- alkyl sulfonates, such as busulfan, etc.;
- nitrosoureas, such as carmustine, lomustine, etc.;
- triazenes, such as dacarbazine, procarbazine, temozolomide, etc.; and
- platinum-containing antineoplastic agents, such as cisplatin, carboplatin and oxaliplatin, which are usually classified as alkylating agents, although they do not alkylate DNA, but cause covalent DNA adducts by a different means, etc.

As used herein, the term "antimetabolite" refers to a chemical that inhibits the use of a metabolite, which is another chemical that is part of normal metabolism. Such substances are often similar in structure to the metabolite that they interfere with, such as the antifolates that interfere with the use of folic acid. The presence of antimetabolites can have toxic effects on cells, such as halting cell growth and cell division, so these compounds are used as chemotherapy for cancer. Anti-metabolites masquerade as a purine or a pyrimidine, preventing their incorporation into DNA during the S phase (of the cell cycle), stopping normal development and division. They also affect RNA synthesis. However, because thymidine is used in DNA but not in RNA (where uracil is used instead), inhibition of thymidine synthesis via thymidylate synthase selectively inhibits DNA synthesis over RNA synthesis. Antimetabolites may be selected from:
- purine analogues, such as azathioprine, mercaptopurine, thioguanine fludarabine pentostatin, cladribine, etc.;
- pyrimidine analogues, such as 5-fluorouracil (5FU), floxuridine (FUDR), cytosine arabinoside (cytarabine), 6-azauracil (6-AU), etc.; or
- antifolates, such as methotrexate, pemetrexed, proguanil, pyrimethamine, trimethoprim, etc.

As used herein, the term "topoisomerase inhibitor" refers to an agent designed to interfere with the action of topoisomerase enzymes (topoisomerase I and II). It is thought that topoisomerase inhibitors block the ligation step of the cell cycle, generating single and double stranded breaks that harm the integrity of the genome. Introduction of these breaks subsequently leads to apoptosis and cell death. Illustrative, non-limitative examples of topoisomerase inhibitors include etoposide, teniposide, topotecan, irinotecan, diflomotecan or elomotecan.

As used herein, the term "anthracycline" refers to a class of drugs (CCNS or cell-cycle non-specific) used in cancer chemotherapy derived from strains of Streptomyces bacteria. Anthracyclines have four mechanisms of action:
1. Inhibition of DNA and RNA synthesis by intercalating between base pairs of the DNA/RNA strand, thus preventing the replication of rapidly-growing cancer cells.
2. Inhibition of topoisomerase II enzyme, preventing the relaxing of supercoiled DNA and thus blocking DNA transcription and replication.
3. Creation of iron-mediated free oxygen radicals that damage the DNA, proteins and cell membranes.
4. Induction of histone eviction from chromatin that deregulates DNA damage response, epigenome and transcriptome.

Illustrative, non-limitative examples of anthracyclines include daunorubicin, doxorubicin, epirubicin, idarubicin, valrubicin, mitoxantrone, etc.

Other anti-cancer agents include, without limitation, the following agents:
- angiogenesis inhibitors, such as angiostatin, endostatin, fumagillin, genistein, minocycline and staurosporin;
- DNA synthesis inhibitors, such as aminopterin, ganciclovir and hydroxyurea;
- enzyme inhibitors, such as S(+)-camptothecin, curcumin, 2-Imino-1-imidazolidineacetic acid (Cyclocreatine), hispidin, formestane, and mevinolin;
- microtubule inhibitors, such as colchicine and dolastatin 15; and
- other anti-tumour agents, such as 17-(allylamino)-17-demethoxygeldanamycin, apigenin, cimetidine, luteinizing hormone-releasing hormone, and pifithrin-α.

In a particular embodiment, the drug is selected from the group consisting of doxorubicin, bevacizumab, capecitabine, cisplatin, cyclophosphamide, epirubicin, 5-fluorouracil, folinic acid, methotrexate, or oxaliplatin.

The agents indicated for the treatment of a particular cancer are common knowledge in the art. By way of an illustrative example, a relation of type of cancers and drugs that are indicated for the treatment of said types of cancers is given in Table 1.

**Table 1**

| **Relation of cancers and drugs that are indicated for their treatment** | |
|---|---|
| **Cancer type** | **Drugs** |
| Breast cancer | Cyclophosphamide, methotrexate, 5-fluorouracil, doxorubicin, bevacizumab |
| Stomach cancer | Epirubicin, cisplatin, 5-fluorouracil, capecitabine |
| Bladder cancer | Methotrexate, vincristine, doxorubicin, cisplatin |
| Lung cancer | Cyclophosphamide, doxorubicin, vincristine, bevacizumab |
| Colorectal cancer | 5-Fluorouracil, folinic acid, oxaliplatin |
| Brain cancer | Bevacizumab |

The pharmaceutical composition of the invention is useful in the treatment of medical conditions, especially for treating tumour diseases or cancers.

### 5. Uses of the particles and compositions of the invention

The particles of the invention may be targeted to a cell, such as a tumor cell (i.e., the target cell), via their targeting moiety, which recognizes and binds to a molecule located in the surface or the target cell. This is of special interest in cancer cells overexpressing cell receptors in the surface; thus, the particles of the invention, which have the ability to recognize a particular cell receptor via their targeting moiety, can be specifically targeted to those tumor cells.

The inventors of the instant invention have synthesized silicon particles comprising an HER2 antibody, which recognizes HER2 positive breast cancer cells. Cell surface receptor recognition by the targeting moiety of the particle is followed by internalization, particle degradation and tumor cell death. Therefore, the particle of the invention, as well as the pharmaceutical composition of the invention comprising a particle of the invention, may be used in the treatment of cancer in a subject in need thereof, wherein the particles of the invention specifically bind to the target cell, by means of interaction and/or binding of the targeting moiety of the particle of the invention to the cell receptor located in the cell surface of the target cell, the particles of the invention are then internalized into the target cell (by any suitable mechanism, for example, a cellular mechanism of endocytosis or similar), the enzymatically metabolizable compound of the internalized particle of the invention is metabolized by the cellular enzymatic machinery, exposing the silica surface of the particle of the invention to the aqueous environment of the target cell, which results in the solubilization of said silica surface and exposition of the silicon body of the particle of the invention to the aqueous environment of the cell. Finally, exposition of the silicon body of the particle of the invention to the cellular aqueous medium causes an explosive oxidation reaction with a high energy yield, causing cell death.

Thus, in a further aspect, the invention relates to the particle of the invention for use in medicine. Alternatively, the invention relates to the use of the particle of the invention for the manufacture of a medicament.

In a further aspect, the invention relates to the particle of the invention for use in the prevention and/or treatment of cancer. Alternatively, the invention relates to the use of the particle of the invention in the manufacture of a medicament for the prevention and/or treatment of cancer. Still alternatively, the invention relates to a method for the prevention and/or treatment of cancer in a subject in need thereof that comprises the administration to said subject of a therapeutically affective amount of particles of the invention.

The term "cancer" has been previously defined and the particulars thereof are incorporated herein by reference. In a particular embodiment, the cancer is selected from the group consisting of breast cancer, colon cancer, gastric cancer, lung cancer, gall bladder cancer, colorectal cancer, bone cancer, sarcoma, esophageal cancer, head and neck carcinomas, ovarian cancer, prostate cancer, liver cancer, melanoma, glioma and neuroblastoma. In a more particular embodiment, the cancer is breast cancer.

As used herein, the term "target cell" refers to the particular cell that internalises the particle of the invention. Upon being internalised, the particle of the invention, through a mechanism that will be explained below, renders a silicon body inside the target cell which explodes and causes the death of the target cell. Any cell can potentially be targeted by the particles of the invention provided that they expose on their surface a molecule which is able to specifically be recognized and bound by the targeting moiety present in the particle of the invention. Illustrative, non-limitative, examples of molecules exposed on the surface of the target cell and able to specifically recognize and/or bind to the targeting moiety of the particle of the invention include cell receptors, cell adhesion molecules, and co-stimulatory molecules. In a particular embodiment, the molecule exposed on the surface of the target cell that is able to specifically be recognized and bound by the targeting moiety of the particle of the invention is a cell receptor, preferably a cell receptor expressed or overexpressed by tumor cells. Cell surface receptors include, without limitation, ion-channel-linked receptors, G-protein-linked receptors, and enzyme-linked receptors. Non-limiting examples of surface cell receptors according to the invention have been previously cited in the context of the particle of the invention. In a particular embodiment, the target cell is a mammalian cell. In a preferred embodiment, the mammalian cell is a human cell. Non-limitative examples of human cells include, without limitation, somatic cells, germ cells and stem cells. Further, in a preferred embodiment, the target cell is a cancer cell or a tumor cell, including a malignant cell. As used herein, the term "tumour cell" or "cancer cell" refers to cells that grow and divide at an unregulated, quickened pace. As the skilled person acknowledges, tumor cells usually overexpress cell surface receptors in comparison with non-tumoral cells. Therefore, in a preferred embodiment, the cell surface receptor exposed on the surface of the target cell that is able to specifically recognize and bind to a targeting moiety of the particle of the invention is a cell receptor which is overexpressed in a tumor cell. For example, breast cancer cells overexpress cell receptors including HER2. In a particular embodiment, the tumor cell is a breast tumor cell, more particularly a breast tumor cell overexpressing HER2.

In a particular embodiment, the particle of the invention for use in the prevention and/or treatment of cancer in a subject in need of treatment exerts its function by a mechanism that comprises killing tumor cells in said subject. Although the inventors do not wish to be bound for any theory, it is believed that the killing of the target (tumor) cells involves a mechanism that comprises:
i. the incorporation of the particle of the invention into the target cell, which involves the targeting moiety of the particle of the invention;
ii. the enzymatic metabolism of the enzymatically metabolizable compound (silicon type I) or of the targeting moiety (silicon type II) of the particle of the invention involving a target cell enzyme, wherein said target cell expresses in its surface a molecule recognizing the targeting moiety of the particle of the invention,
iii. the solution of the silica surface inside the target cell; and
iv. the explosion of the silicon body inside the target cell.

Firstly, the particle of the invention is targeted to a target cell, such as a tumor cell, wherein the targeting involves recognition and/or binding between the targeting moiety of the particle of the invention and a molecule located in the surface of the target cell (such as a cell receptor) which recognizes and/or binds the targeting moiety of the particle of the invention. This recognition and binding of the targeting moiety of the particle of the invention and its corresponding cell receptor is followed by internalization of the particle into the target tumor cell by a suitable mechanism, such as, for example, by a cellular mechanism of endocytosis or similar.

Secondly, and following internalization of the particle of the invention into the target cell, the particle of the invention is exposed to the cellular internal content, particularly to the cellular enzymatic machinery. As it has been previously described, the type I particle of the invention comprises an enzymatically metabolizable compound linked to the silica surface of the particle and linked as well to the targeting moiety of the particle of the invention. Thus, after internalization of the type I particle of the invention, the enzymatically metabolizable compound is exposed to the cellular enzymatic machinery, so said compound is metabolized and degraded by its corresponding specific cell enzyme, wherein said corresponding specific enzyme is any target cell catabolic enzyme whose substrate is the enzymatically metabolizable compound of the particle and results in the degradation of said compound. In a particular embodiment, the enzymatically metabolizable compound of the particle of the invention is selected from the group comprising a carbohydrate, a lipid, a peptide, a protein and a nucleic acid. In a more particular embodiment, the enzymatically metabolizable compound of the particle of the invention is a carbohydrate, more particularly a glucopyranoside or glucopyranoside derivative. In a particular embodiment, the enzymatically metabolizable compound of the particle of the invention is a substrate for a lysosomal enzyme including, without limitation, a lysosomal protease, a lysosomal acid lipase, a lysosomal nuclease and a lysosomal glycoside hydrolase, preferably a lysosomal glycoside hydrolase, more preferably a lysosomal glucohydrolase, even more preferably alpha-D-glucoside glucohydrolase.

Alternatively, in the case of the silicon type II particle of the invention, said particle comprises a targeting moiety which is directly linked to the functionalized silica surface of the particle. After internalization of the type II particle of the invention, the targeting moiety remains exposed to the cellular enzymatic machinery, so said moiety may be metabolized and degraded by a cell enzyme, wherein said enzyme is any target cell catabolic enzyme whose substrate is the targeting moiety of the particle and results in the degradation of said moiety. In a particular embodiment, the targeting moiety is selected from the group comprising an antibody or an antigen binding fragment thereof, a binding structure, an aptamer, a peptide or a small molecule.

Thirdly, following the enzymatic metabolization and degradation of the enzymatically metabolizable compound of the type I particle of the invention, or the metabolization and degradation of the targeting moiety of the type II particle of the invention, the silica surface thereof becomes exposed to the aqueous environment of the cell. As the skilled person knows, silica is highly soluble in water. Therefore, when the silica surface of the particle contacts the aqueous cell environment, it becomes rapidly dissolved, exposing the silicon body (core) of the particle to cell environment.

Finally, the silicon body of the particle of the invention becomes exposed to cell environment. As the skilled person knows, the reaction of silicon and water promotes an explosive oxidation reaction (Clement D et al. 2005 Phys Stat Sol A 202: 1357-1359). Thus, the silicon body reacts with the aqueous environment of the target cell with high energy yield, which results in an explosion causing tumor cell death. As a consequence, this explosive reaction yields soluble biocompatible residues which are easily excretable by urine.

Silicon is characterized by a reduction potential of -1.697 eV to yield silicates or -0.91 eV to yield silica, which latterly are dissolved as silicates, in the presence of water. The low reduction potential makes the reactions violent and even explosive in nanoscaled porous particles. On the other hand, the high tendency of silicon to get oxidized is modulated by the spontaneous generation of a pasivation layer of SiOx at open atmosphere. Noteworthy this pasivation layer is dissolved in water and especially in lightly acidic media. The kinetics of dissolution of this layer can be also modulated by surface functionalization of the silica. Thus, by placing a compact monolayer of an organic molecule, the dissolution process can be retarded or even quenched.

The invention is described in detail below by means of the following examples which are to be construed as merely illustrative and not limitative of the scope of the invention.

### EXAMPLE 1

### Synthesis of silicon particles

### 1. Materials and Methods

### 1.1 Synthesis of porous silicon particles

The method for obtaining porous silicon microspheres is based on the decomposition of disilane gas (Si₂H₆) by means of chemical vapor deposition (CVD). It is similar to the synthesis procedure of silicon colloids, where the gas is introduced in a reactor whose walls are heated at high temperatures for a certain time, usually higher than 400°C. During this procedure, SiₙHₘ clusters grow in the gas phase and they become highly spherical micrometer size particles thanks to surface tension forces. At the same time, there is a hydrogen desorption process from the clusters that makes the hydrogen content decrease progressively until they become hydrogenated amorphous silicon (a:Si-H) colloids. In order to obtain porous silicon microspheres, the heating process is stopped at an early stage, before the formation of amorphous silicon colloids has finished. In this way, porous particles with an undetermined composition of silicon and hydrogen atoms are obtained.

Porous silicon microspheres were synthesized by using a low temperature for decomposing disilane, namely 400°C, rather than higher temperatures because this allowed an optimal control of the process timing. The absolute gas pressure in the reactor was about 130 kPa at room temperature, and decomposition times (DT) from 1 to 2 minutes were used. Since it takes several minutes for the gas to reach the desired temperature and start decomposing, the path of a He-Ne laser was monitored through the reactor, allowing the control of particle formation start. The laser path can actually be seen inside the reactor when floating particles exist in the gas even although such particles are much smaller than the wavelength of the laser, i.e. 613 nm, by virtue of Rayleigh scattering. This time is taken as the reference starting point to measure DT.

### Synthesis of 2-acetamido-2-deoxy-β-d-glucopyranosyloxyacetic acid

Solvents were dried according to known methods and distilled before use. All other reagents were commercial compounds of the highest purity available. Unless otherwise indicated, all reactions involving air- and moisture-sensitive materials were carried out under argon atmosphere, and those not involving aqueous reagents were carried out in oven-dried glassware. Analytical thin layer chromatography (TLC) was performed on aluminum plates with Merck Kieselgel 60F254 and visualized by UV irradiation (254 nm) or by staining with an ethanolic solution of phosphomolibdic acid. Flash column chromatography was carried out using Merck Kieselgel 60 (230-400 mesh) under pressure. ¹H NMR spectra were recorded in CDCl₃ and D₂O, at ambient temperature on AMX-400 spectrometer at 400 MHz, with residual protic solvent as the internal reference [CDCl₃, δ_{H} = 7.26 ppm]; chemical shifts (δ) are given in parts per million (ppm), and coupling constants (*J*) are given in Hertz (Hz). The proton spectra are reported as follows: δ (multiplicity, coupling constant J, number of protons, assignment).

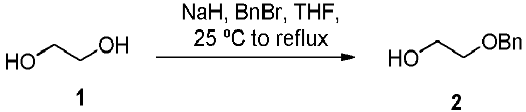

### 2-(Benzyloxy)ethanol

To a mixture of NaH (3.4 g, 0.085 mol, 60% w/w in mineral oil) in THF (150 mL), ethylene glycol (1) (25.1 mL, 0.45 mol) was added and the mixture was stirred for 1 h at 25°C. Then, benzyl bromide (8.9 g, 0.075 mol) was added and the reaction was refluxed for 12 h. After cooling down the mixture (0°C), a saturated aqueous solution of NH₄Cl was added, the solvent was evaporated, and the mixture was extracted with EtOAc (3x). The combined organic layers were washed with a saturated aqueous solution of NH₄Cl and brine, and dried (Na₂SO₄). The solvent was evaporated to afford 11.22 g (98% yield) of a colorless oil identified as 2-(benzyloxy)ethanol (2). The spectroscopic data were identical to those described in the literature. ¹H-NMR (400 MHz, CDCl₃): δ 7.40-7.30 (m, 5H), 4.58 (s, 2H), 3.78 (t, *J=* 5.0 Hz, 2H), 3.61 (t, *J=* 4.9 Hz, 2H), 2.06 (br, 1H, OH) ppm.

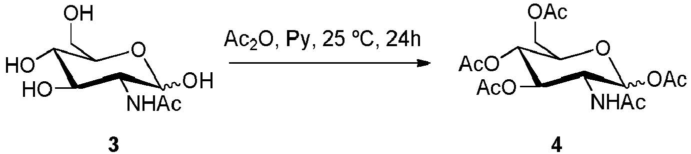

### 1,3,4,6-tetra-O-acetyl-α/β-N-acetylglucosamine

N-acetyl-D-glucosamine (3) (5 g, 22.6 mmol) was dissolved in pyridine (36 mL) and acetic anhydride (25 mL) was added dropwise at 0°C. The reaction mixture was stirred at 25°C for 24 h, then diluted with CH₂Cl₂ and washed consecutively with cold water, a saturated aqueous solution of NaHCO₃, and a 10% aqueous solution of CuSO₄. The organic layer was dried (Na₂SO₄) and the solvent was evapotared to obtain 6.60 g (75%) of a white solid identified as 1,3,4,6-tetra-O-acetyl-α/*β*-*N*-acetylglucosamine (4). The spectroscopic data were identical to those described in the literature. ¹H-NMR (400 MHz, CDCl₃): δ 6.17 (d, J= 3.6 Hz, 1H), 5.64 (d, J= 9.3 Hz, 1H), 5.30-5.20 (m, 2H), 4.49 (ddd, J= 10.6, 8.9, 3.6 Hz, 1H), 4.25 (dd, J= 12.5, 4.1 Hz, 1H), 4.07 (dd, J= 12.5, 2.4 Hz, 1H), 4.00 (ddd, J= 9.6, 4.0, 2.3 Hz, 1H), 2.20 (s, 3H), 2.09 (s, 3H), 2.06 (s, 3H), 2.05 (s, 3H), 1.94 (s, 3H) ppm.

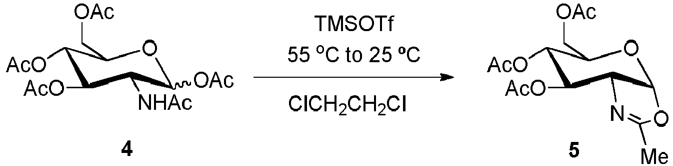

### 4',5'-Dihydro-2'-methyloxazolo[5',4':1,2]-3,4,6-tri-O-acety/-1,2-dideoxy-α-D glucopyranoside

To a solution of 1,3,4,6-tetra-*O*-acetyl-α/β-*N*-acetylglucosamine (4) (2.49 g, 6.4 mmol) in dichloroethane (25 mL) TMSOTf (1.8 mL, 9.6 mmol) was added and the reaction was stirred for 2 h at 55 °C and for 12 h at 25°C. A saturated aqueous solution of NaHCO₃ was added and the mixture was extracted with CH₂Cl₂ (3x). The combined organic layers were washed with a saturated aqueous solution of NaHCO₃ and dried (Na₂SO₄) and the solvent was evaporated. The resulting residue was purified by column chromathography (silicagel, 97:3 CH₂Cl₂/MeOH) to afford 1.86 g (78%) of 4',5'-dihydro-2'-methyloxazolo[5',4':1,2]-3,4,6-tri-(3-acetyl-1,2-dideoxy-α-D-glucopyranoside (5). The spectroscopic data were identical to those described in the literature. ¹H-NMR (400 MHz, CDCl₃): δ 5.98 (d, *J*= 7.4 Hz, 1H), 5.27 (t, *J*= 2.4 Hz, 1H), 4.94 (ddd, J= 9.2, 2.0, 1.2 Hz, 1H), 4.19-4.14 (m, 3H), 3.62 (dt, *J*= 8.8, 4.3 Hz, 1H), 2.12 Hz (s, 3H), 2.11 (s, 3H), 2.10 (s, 3H), 2.09 (s, 3H) ppm.

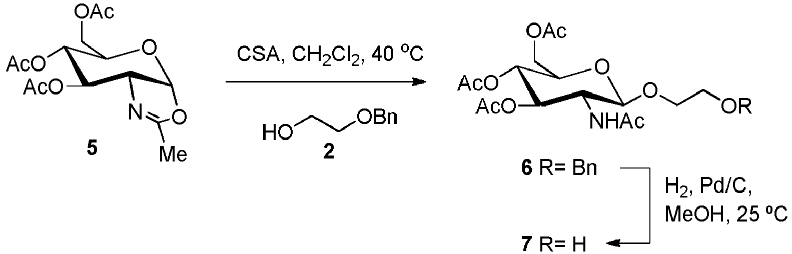

### 2-Hydroxyethyl-2-acetamido-3,4,6-tri-O-acetyl-2-deoxy-β-D-glucopyranoside

10-(*R*)-camphorsulfonic acid (1.40 g, 5.02 mmol) and 2-(benzyloxy)ethanol (2) (7.63 g, 50.2 mmol) were added to a stirred solution of 4', 5'-dihydro-2'-methyloxazolo[5',4':1,2]-3,4,6-tri-*O*-acetyl-1,2-dideoxy-α-D-glucopyranoside (5) (1.86 g, 5.02 mmol) and powdered 4 Å molecular sieves (ca. 8 g) in CH₂Cl₂ (30 mL) and the reaction was stirred at 40 °C for 14 h. The mixture was cooled down to 0°C, and a saturated aqueous solution of NaHCO₃ (60 mL) and CH₂Cl₂ (30 mL) were added. The layers were separated and the organic layer was washed with a saturated aqueous solution of NaHCO₃, brine and dried (Na₂SO₄), and the solvent was evaporated. The residue was purified by column chromatography (hexane/EtOAc 50:50 to CH₂Cl₂/MeOH 95:5) providing 2-*O*-benzyloxyethyl-2-acetamido-3,4,6-tri-O-acetyl-2-deoxy-β-D-glucopyranoside (6) as a colorless solid. The spectroscopic data of the product were identical to those described in the literature. ¹H-NMR (400 MHz, CDCl₃): δ 7.4-7.3 (m, 5H), 5.50 (d, *J=* 8.8 Hz, 1H), 5.25 (dd, *J=* 10.4, 9.4 Hz, 1H), 5.09 (t, *J=* 9.6 Hz, 1H), 4.76 (d, *J=* 8.4 Hz, 1H), 4.56 (app s, 2H), 4.27 (dd, *J=* 12.3, 4.7 Hz, 1H), 4.20-4.15 (m, 2H), 3.99 (dt, *J=* 11.5, 3.9 Hz, 1H), 3.80 (ddd, *J=* 18.2, 14.7, 11.2 Hz, 1H), 3.70-3.65 (m, 3H), 2.09 (s, 3H), 2.06 (s, 3H), 2.03 (s, 3H), 1.87 (s, 3H) ppm.

A mixture of 2-*O*-benzyloxyethyl-2-acetamido-3,4,6-tri-*O*-acetyl-2-deoxy-*β*-D-glucopyranoside (6) (0.19 g, 0.36 mmol) and Pd/C (10%, 0.02 g) in MeOH (3 mL) was stirred under H₂ atmosphere for 7 h at 25°C. The reaction was filtered through Celite and the solvent was evaporated to afford 0.15 g (96%) of a solid identified as 2-hydroxyethyl-2-acetamido-3,4,6-tri-*O*-acetyl-2-deoxy-β-D-glucopyranoside (7). The spectroscopic data of the product were identical to those described in the literature. ¹H-NMR (400 MHz, CDCl₃): δ 5.58 (d, *J=* 9.0 Hz, 1H), 5.25 (dd, *J=* 10.6, 9.4 Hz, 1H), 5.07 (t, *J=* 9.6 Hz, 1H), 4.71 (d, *J=* 8.3 Hz, 1H), 4.2-4.10 (m, 2H), 3.9-3.8 (m, 2H), 3.7-3.6 (m, 2H), 2.58 (br, 1H, OH), 2.11 (s, 3H), 2.06 (s, 3H), 2.05 (s, 3H), 1.98 (s, 3H) ppm.

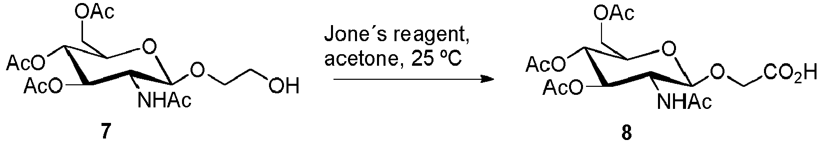

### 2-acetamido-3,4,6-tri-O-acetyl-2-deoxy-β-D-glucopyranosyloxyacetic acid

Jones reagent (3.5 M, 0.2 mL, 0.69 mmol) was added to a stirred solution of 2-hydroxyethyl-2-acetamido-3,4,6-tri-*O*-acetyl-2-deoxy-β-D-glucopyranoside (7) (0.15 g, 0.35 mmol) in acetone (2 mL) at 0 °C and the reaction was stirred for 13 h at 25°C. Isopropanol was added and the solvent was evaporated. After the addition of CH₂Cl₂ (10 mL) and brine (10 mL), the layers were separated, the organic layer was washed with brine and dried (Na₂SO₄), and the solvent was evaporated to afford 0.11 g (73%) of a white solid that was identify as 2-acetamido-3,4,6-tri-*O*-acetyl-2-deoxy-β-D-glucopyranosyloxyacetic acid (8). The spectroscopic data of the product were identical to those described in the literature. ¹H-NMR (400 MHz, CDCl₃): δ 6.57 (br s, 1H), 5.22 (t, *J=* 9.6 Hz, 1H), 5.11 (t, *J=* 9.6 Hz, 1H), 4.75 (d, *J=* 8.1 Hz, 1H), 4.35 (app s, 2H), 4.28 (dd, *J=* 12.1, 4.9 Hz, 1H), 4.15 (dd, *J=* 10.7, 2.1 Hz, 1H), 4.1-4.0 (m, 1H), 3.7-3.6 (m, 1H), 2.11 (s, 3H), 2.06 (s, 3H), 2.03 (s, 3H), 1.97 (s, 3H) ppm.

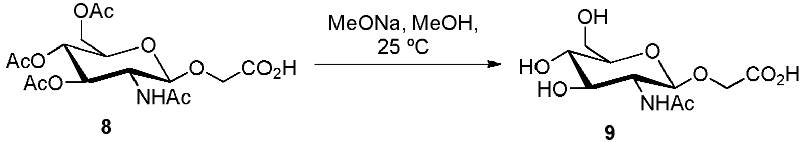

### 2-acetamido-2-deoxy-β-D-glucopyranosyloxyacetic acid

Sodium methoxide (0.02 g, 0.332 mmol) was added to a stirred solution of 2-acetamido-3,4,6-tri-*O*-acetyl-2-deoxy-β-D-glucopyranosyloxyacetic acid (8) (0.114 g, 0.255 mmol) in MeOH (6 mL) and the reaction mixture was stirred at 25°C for 24 h. After the addition of Dowex-200 the mixture was filtered and the solvent was evaporated. The residue was dissolved in H₂O and dried under vacuum to afford 0.071 g (99%) of a solid identified as 2-acetamido-2-deoxy-β-D-glucopyranosyloxyacetic acid (9). The spectroscopic data of the product were identical to those described in the literature. ¹H-NMR (400 MHz, D₂O): δ 4.49 (d, *J*= 8.4 Hz, 1H), 4.18 (s, 2H), 3.83 (d, *J=* 12.2 Hz, 1H), 3.70-3.60 (m, 2H), 3.50-3.40 (m, 1H), 3.40-3.30 (m, 2H), 1.97 (s, 3H) ppm.

### 1.2 Particle Functionalization

A suspension of APS-coated silica particles was produced by treatment of 0.1 g silica particles with aminopropylsilane (APS, 20 µL) in 2-propanol (5 mL) at 80 °C for 2 h. The beads were centrifuged at 3800 rpm for 30 min to remove the excess APS, followed by replacement of the supernatant solution by isopropanol. The particles were re-dispersed by shaking (ultrasound) for 10 min. This protocol was repeated two more times.

The beads were centrifuged at 3800 rpm for 30 min and the supernatant was replaced by dimethylformamide (DMF). Then, a solution of 2-acetamido-2-deoxy-β-D-glucopyranosyloxyacetic acid **(9)** (14 mg), BOP [benzotriazol-1-yloxy)tris(dimethylamino)phosphonium hexafluorophosphate, 29 mg], hydroxybenzotriazole (HOBt, 7 mg) in dimethylformamide (DMF, 3 mL) and diisopropylethylamine (30 µL) were added and the suspension was stirred for 12 h at 25°C. The beads were centrifuged at 3800 rpm for 30 min and washed with DMF and the supernatant was replaced by H₂O. The particles were re-dispersed by shaking (ultrasound) for 10 min. The supernatant was replaced by phoshate buffered saline (PBS, 1x).

To a suspension of the particles in PBS (2 mL), 250 µL of a solution of anti-erbB-2/HER2 (1 µL/L in PBS) was added and the mixture was shaken at 0°C for 12 h. The beads were centrifuged at 3800 rpm for 10 min, and the supernatant was replaced by PBS. The particles were re-dispersed by shaking (ultrasound) for 3 min at 0°C.

### 2. Results

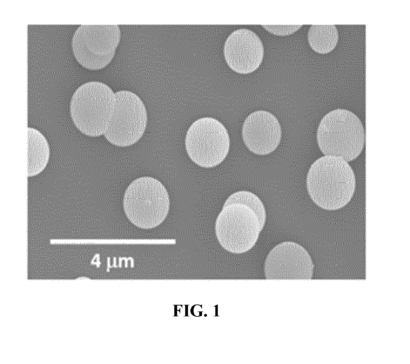

The particles obtained according to the previously disclosed process, i.e., an illustrative example of silicon particle of the present invention, are highly spherical with a diameter that may range from the nano- to the micro- scale (Fig. 1). As prepared and after extracting the materials from the reactor, the exposition to the atmospheric oxygen generates a thin layer of silicon oxide. This coating acts as a protective shell, which also generates an easily functionalizable surface to couple an organic layer that will protect the silica from dissolution in physiological media. This protective organic shell should be easily degradable by the enzymatic machinery present in the cell. To prove this concept, the inventors have used glucopyranoside, a well-known molecule, which can be easily metabolized by the lysosomal enzyme α-D-glucoside glucohydrolase. The lysosome has been shown to be a target organelle for most particles.

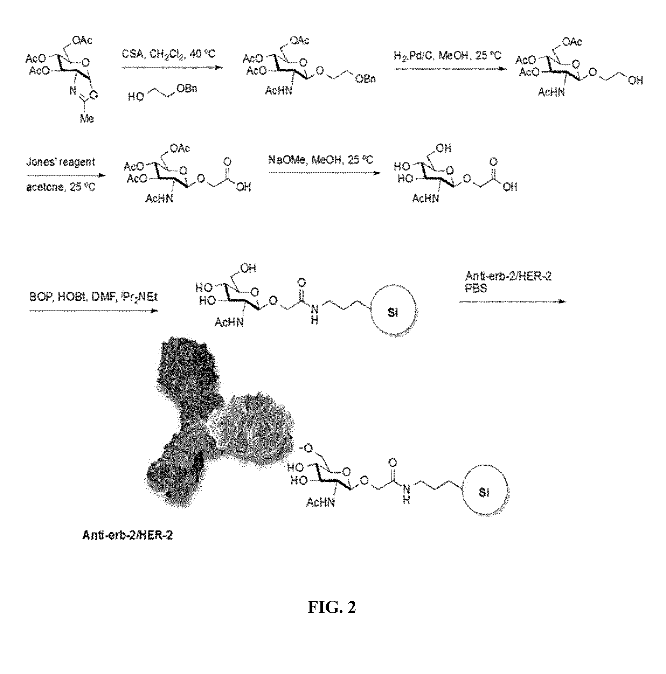

The sugar and the particle cannot be directly attached without previous modifications. Thus, the silicon particles (SiPs) were capped with aminopropylsilane (APS) while the glucopyranoside selected was 2-acetamido-2-deoxy-β-D-glucopyranosyloxyacetic acid (Fig. 2). The BOP/HOBt coupling method (BOP: benzotriazol-1-yl-oxy-tris (dimethylamino) phosphonium hexafluorophosphate; HOBt: 1-hydroxybenzotriazole) was chosen to generate a peptide bond between the amine-functionalized particle surface and the carboxylic acid group attached to the carbohydrate. Notably, within this configuration, the hybrid particles resist oxidation in physiological media, but could be degraded unselectively within any cell following endocytosis. Therefore, for anticancer therapy a selective antibody that targets the surface receptor of the desired cell would be necessary.

Consequently, a further step in the preparation of the immunotherapeutic material of the invention implies the coupling of a directing vector. The HER2-positive breast cancer is characterized by the amplification of this gene and its protein high expression and activity. In fact, there is a strong association between HER2 (ErbB2) tyrosine kinase expression and the aggressiveness and prognosis of the disease. Fortunately, HER2 amplification confers a selective target for a specific treatment. In fact, several drugs targeting his receptor are actually used for breast cancer treatment in medical practice, including small molecule tyrosine kinase inhibitors (TKIs) such as gefitinib, erlotinib or lapatinib or monoclonal antibodies such as trastuzumab, pertuzumab or cetuximab. Rather than using standard carbodiimide chemistry, the HER2 antibody was attached to the particles by taking advantage of its affinity for the sugar. Thus, one of the four glycosilation immunogenic regions was spontaneously coupled to the sugars in the particle allowing the other three to interact with the cell membrane receptors.

### EXAMPLE 2

### Activity of silicon particles

The efficiency of the SiPs functionalized with antibodies against HER2 receptors (SiPs-HER2), obtained in Example 1, for their potential for selectively killing only HER2 overexpressing cells was analyzed. To this end, the inventors used two different cellular lines, one overexpressing the HER2 receptor (SK-BR-3) and the other one with its normal expression level (MDA-MB-435). The cells were seeded in 96 well plates and incubated with different quantities of SiPs-HER2 for 48 h. Then a resazurin-based viability assay was performed.

### 2.1 Cell culture and viability assay

### Materials

Dulbecco's modified eagle's medium (DMEM, #30-2002) and McCoy's 5a medium (#30-2007) were purchased from ATCC. Fetal bovine serum (FBS, #S0615) was obtained from Biochrom AG and penicillin/streptomycin (#15140-122) from Gibco (#15140-122). L-glutamine (#25030-024) was purchased from Life Technologies. The viability assay based on Resazurin (#TOX8) and the 96 well plates (#CLS3603) in which the viability assay was carried out were obtained from Sigma-Aldrich.

MDA-MB-435 human epithelial cells (ATCC #HTB-129) were seeded and grown in growth medium (DMEM with 4.5 g/L glucose supplemented with 10% FBS, 1% L-glutamine (200 mM) and 1% penicillin/streptomycin. SK-BR-3 human breast adenocarcinoma cells (ATCC #HTB-30) were seeded and grown in another growth medium (McCoy's medium supplemented with 10% FBS, 1% L-glutamine (200 mM) and 1% penicillin/streptomycin.

### Viability assay

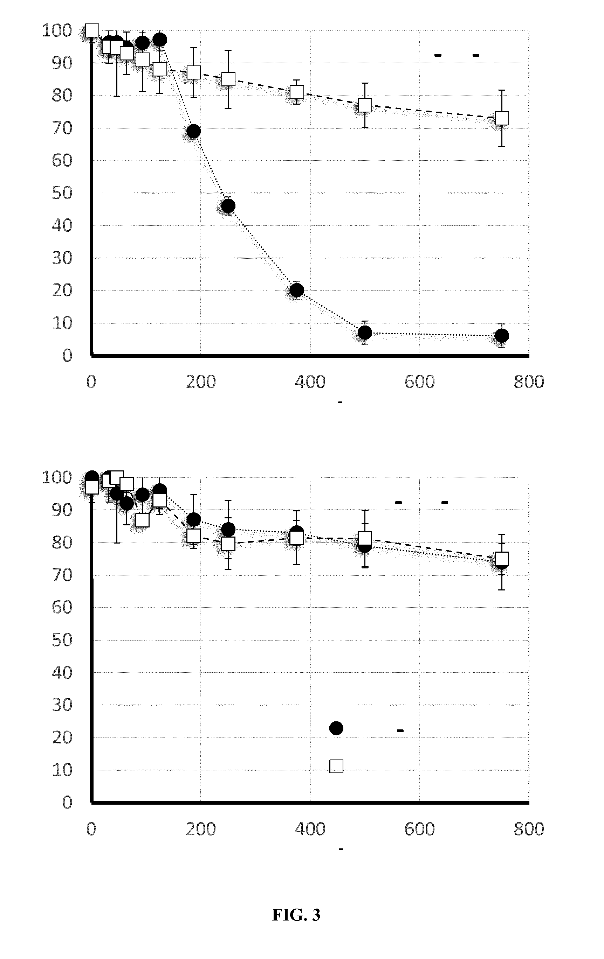

20,000 cells (MDA-MB-435 or SK-BR-3) were seeded per well in a 96 well plate and incubated in 100 µL of the corresponding cell medium for 48 h at 37°C and 5% CO₂. After this time, different concentrations of particles (SiPs or SiPs-HER2) were added and cells were incubated for another 48 h. For each concentration the viability assay was repeated three times. Control viability assays were performed with cells without particles, and with particles without cells. After incubation, cellular viability was probed. Cells were washed with PBS and a solution of 10% of resazurin in growth medium was added to each well. Cells were placed in the incubator for 3 h (37°C and 5% CO₂). Resazurin is a nonfluorescent molecule which is reduced from the oxidized to the reduced form called resofurin by metabolically active cells. Resofurin is fluorescent, has a maximum emission wavelength at 585 nm (red emission), and can be excited from 530 to 560 nm. The fluorescent emission intensity originating from resofurin is proportional to the number of metabolically active (that is, living) cells. Fluorescence emission was measured with a Fluorolog-3 spectrofluorometer equipped with a microwell plate reader (MicroMax 384) from Horiba JOBIN YVON. The samples were excited at 560 nm and the emission spectra were collected from 572 nm to 650 nm. Background was subtracted from the spectra. As the position of the maximum emission wavelength can be slightly shifted the peak emission was averaged from 584 nm - 586 nm. The emission peak intensity values were normalized, considering a cell viability of 100% for the control experiments in which no particles has been added to the cells. The normalized fluorescence emission peak intensities were plotted against the logarithm of the particle concentration (Fig. 3). Dose-response curves were obtained for SK-BR-3 and MDA-MB-435 exposed to different concentrations of SiPs and SiPs-HER2 (Fig. 3). The results were fitted to sigmoidal curves and the inflexion point was calculated. The inflexion point represents the LD₅₀ value, which in this case is the concentration of particles at which cell viability is reduced to 50%, i.e. 50% of the cells are no longer metabolically active. The calculated LD₅₀ values are shown in Table 2.

### Results

The efficiency of the SiPs functionalized with antibodies against HER2 receptors (SiPs-HER2) for their potential for selectively killing only HER2 overexpressing cells was analyzed. To this end, the inventors used two different cellular lines, one overexpressing the HER2 receptor (SK-BR-3) and one with its normal expression level (MDA-MB-435). The cells were seeded in 96 well plates and incubated with different quantities of SiPs-HER2 for 48 h. Then a resazurin-based viability assay was performed. Resazurin is a nonfluorescent molecule that is reduced by metabolic active cells to the fluorescent resorufin. Thus, the number of viable cells can be determined in terms of resorufin fluorescence. Notably, when SK-BR-3 cells (cells overexpressing the HER2 receptors) were treated with SiPs-HER2 their viability was clearly compromised (Fig. 3a). On the other hand SK-BR-3 cells treated with PSiPs (Fig. 3a) or MDA-MB-435 cells treated with SiPs or SiPs-HER2 (Fig. 3b) showed a more delayed toxicological response.

The LD50 (lethal dose killing 50% of the cell population) values are presented in Table 2. Only 249 µg/mL SiPs-HER2 were needed to kill 50% of the SK-BR-3 cells, whereas much higher quantities of the same particles were required to kill under the same conditions the same amount of MDA-MB-435 cells. On the other hand, SiPs without attached HER2 antibodies showed a much lower toxicological response, as their LD₅₀ values were very high for both cell types.

**Table 2**

| **Calculated LD₅₀ obtained from the dose-response curves as shown in** **Figure 3** | | |
|---|---|---|
| **Cell line** | **Type of NP** | **LD₅₀ (µg/mL)** |
| SK-BR-3 | PSiPs-HER2 | 249 |
| MDA-MB-435 | PSiPs-HER2 | 3776 |
| SK-BR-3 | PSiPs | 5603 |
| MDA-MB-435 | PSiPs | 4672 |

These results confirm the efficacy of SiPs-HER2 to selectively recognize the HER2 receptors present on the surface of SK-BR-3 cells and to effectively promote local accumulation. The degree of targeting was more than sufficient to accelerate the death of the targeted cancer cells.

The particles described herein represent an immunotherapy approach for potential cancer treatment. This platform comprises the use of engineered silicon particles conjugated with a selective antibody. The conceptual advantage of the system described herein is that after reaction, the particles are degraded to soluble and excretable biocomponents. In particular, the present invention shows the specific targeting of cancer cells *in vitro.* The fact that the LD₅₀ value of SiPs-HER2 tumoral cells is 15 fold lower than the LD50 value for non-tumoral cells demonstrates very high specificity *in vitro.* The present invention represents a first important step on the long road to be continued towards the design of a new potential chemotherapy agent against cancer in general and breast cancer in particular.

## Claims

1. A silicon particle selected from the group consisting of a type I silicon particle and a type II silicon particle,
wherein the type I silicon particle comprises:
a. a silicon body,
b. a silica surface surrounding the silicon body, wherein the silica surface is functionalized by at least one functional group, and wherein said functional group is capable of setting up a bond with an enzymatically metabolizable compound,
c. an enzymatically metabolizable compound, wherein said compound is linked to (i) said silica surface through said functional group and to (ii) a targeting moiety, and
d. a targeting moiety, wherein said moiety is linked to the particle through said enzymatically metabolizable compound, and
wherein the type II silicon particle comprises:
a. a silicon body,
b. a silica surface surrounding the silicon body, wherein the silica surface is functionalized by at least one functional group, and wherein said functional group is capable of setting up a bond with a targeting moiety, and
c. a targeting moiety, wherein said moiety is directly linked to said functionalized silica surface.

2. The particle according to claim 1, wherein said silica surface is functionalized by reaction with a compound selected from the group comprising an halosilane, an organosilane, a silanol, a siloxane, an aminosilane, a mercaptosilane and a glycidoxysilane.

3. The particle according to claim 1 or 2, wherein said silica surface is functionalized by reaction with aminopropylsilane (APS).

4. The particle according to any one of claims 1 to 3, wherein said enzymatically metabolizable compound is selected from the group comprising a carbohydrate, a lipid, a peptide, a protein and a nucleic acid.

5. The particle according to claim 4, wherein said enzymatically metabolizable carbohydrate is a glucopyranoside.

6. The particle according to any one of claims 1 to 5, wherein said targeting moiety is a molecule able to specifically recognize and bind to a cell receptor of a target cell, wherein said targeting moiety is selected from the group comprising an antibody or an antigen binding fragment thereof, a binding structure, an aptamer, a peptide or a small molecule.

7. A process for the preparation of a particle according to any of claims 1 to 6, selected from the group consisting of "Process I" and "Process II",
- wherein Process I comprises:
i) contacting a silicon particle comprising a silicon body and a functionalized silica surface with an enzymatically metabolizable compound under conditions suitable for binding the compound to the functionalized silica surface; and
ii) contacting the product resulting from step i) with a targeting moiety under conditions suitable for binding the targeting moiety to the compound bound to the functionalized silica surface, and
- wherein Process II comprises contacting a silicon particle comprising a silicon body and a functionalized silica surface with a targeting moiety under conditions suitable for binding the targeting moiety to the functionalized silica surface.

8. A composition comprising at least a particle according to any of claims 1 to 6 and a suitable medium.

9. A pharmaceutical composition comprising a therapeutically effective amount of a particle according to any of claims 1 to 6 and a pharmaceutically acceptable excipient.

10. The pharmaceutical composition according to claim 9, which further comprises an anti-cancer agent.

11. The particle according to any of claims 1 to 6, or the pharmaceutical composition according to claim 9 or 10, for use in medicine.

12. The particle according to any of claims 1 to 6 for use in the prevention and/or treatment of cancer.

13. The particle for use according to claim 12, wherein prevention and/or treatment of cancer comprises killing tumor cells.

14. The particle for use according to claim 12 or 13, wherein the cancer is selected from the group consisting of breast cancer, colon cancer, gastric cancer, lung cancer, gall bladder cancer, colorectal cancer, bone cancer, sarcoma, esophageal cancer, head and neck carcinomas, ovarian cancer, prostate cancer, liver cancer, melanoma, glioma and neuroblastoma.
